# EUROPEAN PATENT SPECIFICATION

(11) **EP 1 729 768 B1**
(45) Date of publication and mention of the grant of the patent: **10.01.2018**
(21) Application number: 05725623.2
(22) Date of filing: 14.03.2005
(51) Int. Cl.: A61K 31/44, A61K 9/107

(54) **IMMUNE RESPONSE MODIFIER FORMULATIONS AND METHODS**
FORMULIERUNGEN UND VERFAHREN ZUR MODIFIZIERUNG DER IMMUNANTWORT
FORMULATIONS MODIFICATRICES DE REPONSE IMMUNITAIRE ET PROCEDES CORRESPONDANTS

(30) Priority: 15.03.2004 US 553148 P
(43) Date of publication of application: 13.12.2006
(73) Proprietor: Meda AB, 170 09 Solna (SE)
(72) Inventor: BUSCH, Terri F.,, Saint Paul, MN 55133-3427 (US); KUEPER, Leo W., III, Saint Paul, MN 55133-3427 (US)
(74) Representative: FRKelly
(86) International application number: PCT/US2005/008576
(87) International publication number: WO 2005/089317

(56) References cited:
- WO-A1-00/26217
- WO-A1-03/045391
- US-A- 5 238 944
- US-B1- 6 194 425
- US-B1- 6 245 776
- CHOLLET J L ET AL: "DEVELOPMENT OF A TOPICALLY ACTIVE IMIQUIMOD FORMULATION", PHARMACEUTICAL DEVELOPMENT AND TECHNOLOGY, NEW YORK, NY, US, vol. 4, no. 1, 1 January 1999 (1999-01-01) , pages 35-43, XP000900717, ISSN: 1083-7450, DOI: 10.1081/PDT-100101337

## Description

### BACKGROUND

There has been a major effort in recent years to find compounds that modulate the immune system. Examples of such compounds, which have demonstrated cytokine inducing and immunomodulating activity, are disclosed in U.S. Patent Nos. 4,689,338; 4,929,624; 5,266,575; 5,268,376; 5,346,905; 5,352,784; 5,389,640; 5,446,153; 5,482,936; 5,756,747; 6,110,929; 6,194,425; 6,331,539; 6,376,669; 6,451,810; 6,525,064; 6,541,485; 6,545,016; 6,545,017; 6,573,273; 6,656,938; 6,660,735; 6,660,747; 6,664,260; 6,664,264; 6,664,265; 6,667,312; 6,670,372; 6,677,347; 6,677,348; 6,677,349; 6,683,088; 6,756,382; 6,797,718; and 6,818,650; and U.S. Patent Publication Nos. 2004/0091491; 2004/0147543; and 2004/0176367. Many of these compounds, also known as immune response modifiers (IRMs), are small organic molecules, for example, imidazoquinoline amine derivatives (see, e.g., U.S. 4,689,338), but a number of other compound classes are known as well (see, e.g., U.S. 5,446,153), and more are still being discovered.

Pharmaceutical formulations containing IRM compounds are disclosed in U.S. Patent Nos. 5,238,944; 5,939,090; and 6,425,776; European Patent 0 394 026; and U.S. Patent Publication 2003/0199538. Many such formulations include preservatives such as methylparaben, sorbic acid, propylene glycol, etc.

The mechanism for the antiviral and antitumor activity of these IRM compounds is thought to be due in substantial part to enhancement of the immune response by induction of various important cytokines (e.g., interferons, interleukins, tumor necrosis factor, etc.). Such compounds have been shown to stimulate a rapid release of certain monocyte/macrophage-derived cytokines and are also capable of stimulating B cells to secrete antibodies, which play an important role in these IRM compounds' antiviral and antitumor activities. One of the predominant immunostimulating responses to these compounds is the induction of interferon (IFN)-α production, which is believed to be very important in the acute antiviral and antitumor activities seen. Moreover, up regulation of other cytokines, such as, for example, tumor necrosis factor (TNF), Interleukin-1 (IL-1) and IL-6 also have potentially beneficial activities and are believed to contribute to the antiviral and antitumor properties of these compounds.

Accordingly, in view of their importance and potential benefit, there is a continuing need for new formulations of these unique compounds.

### SUMMARY OF THE INVENTION

According to the present invention topical formulations according to any of claims 1 to 12 are provided.

Although some of the beneficial effects of IRMs are known, the ability to provide therapeutic benefit via topical application of an IRM compound for treatment of a particular condition at a particular location may be hindered by a variety of factors. These factors include, e.g., irritation of the skin to which the formulation is applied; formulation wash away; insolubility and/or degradation of the IRM compound in the formulation; physical instability of the formulation (e.g., separation of components, thickening, precipitation/agglomeration of active ingredient, and the like); degradation of excipients; poor permeation; and undesired systemic delivery of the topically applied IRM compound.

It has now been found that formulations of IRM compounds containing a 2-aminopyridine moiety fused to a five-membered nitrogen- containing heterocyclic ring in combination with sorbic acid may suffer impaired stability of both the IRM compound and the sorbic acid. However, it has further been found that addition of a pharmaceutically acceptable antioxidant compound to the formulation can reduce degradation of the IRM compound and sorbic acid, thereby providing improved stability. Sorbic acid and related salts and esters are often used as preservative systems and can be particularly suitable for use in a multi-dose dispenser formulation (see, for example, U.S. Patent No. 6,245,776 (Skwierczynski et al.)), but stability is an important issue for formulations and can reduce shelf life of a product or even jeopardize regulatory approvability.

In particular, it appears that IRMs containing a 2-aminopyridine moiety fused to a five-membered nitrogen- containing heterocyclic ring interact with preservatives such as sorbic acid, resulting in decreased concentrations (relative to the initial concentrations in the freshly prepared formulation) of both the IRM and preservative, with the resulting formation of unwanted impurities. Although not intending to be bound to any particular theory or mechanism, it is hypothesized that these IRMs interact with intermediates and products resulting from autoxidation of the sorbic acid preservative.

It has been discovered that stability can be improved through the addition of a compound acting as as antioxidant. The antioxidant may beneficially have hydrogen atom donating functionality. Moreover, when an antioxidant compound is included with the IRM compound and sorbic acid, stability of the formulation may be further improved by adding a chelating agent.

In one embodiment, the present invention provides a topical formulation that includes: an immune response modifier (IRM) compound which is an imidazonaphthyridine amine; a preservative system that includes sorbic acid, esters thereof, salts thereof, or combinations thereof; and an antioxidant.

A chelating agent may also beneficially be included in any of the formulations described herein. Furthermore, a fatty acid, a hydrophobic, aprotic component miscible with the fatty acid and including a hydrocarbyl group of 7 or more carbon atoms, or combinations thereof, may also be included in any of the formulations described herein.

For example, in another embodiment, the present invention provides a topical formulation that includes: an immune response modifier (IRM) compound which is an imidazonaphthyridine amine; a preservative system that includes a sorbic acid preservative selected from the group consisting of sorbic acid, esters thereof, salts thereof, and combinations thereof; an antioxidant having hydrogen atom donating functionality; a fatty acid; and a hydrophobic, aprotic component miscible with the fatty acid and having a hydrocarbyl group of 7 or more carbon atoms. A chelating agent may also beneficially be included.

In another embodiment, the present invention provides a topical formulation that includes: 0.001% by weight to 5.0% by weight of an immune response modifier (IRM) compound which is an imidazonaphthyridine amine (and preferably 2-methyl-1-(2-methylpropyl)-1*H-*imidazo[4,5-*c*][1,5]naphthyridin-4-amine); a preservative system; 0.001% by weight to 0.2% by weight of an antioxidant having hydrogen atom donating functionality; 0 to 0.1% by weight of a chelating agent; 1 % by weight to 30% by weight of a fatty acid; 1 % by weight to 15% by weight of a medium-chain triglyceride; 0.2% by weight to 2.0% by weight of a viscosity enhancing agent; 0.1% by weight to 6.0% by weight of an emulsifier; and water; wherein the formulation has a pH of 4.0 to 6.0 and the weight percentages are based on the total weight of the formulation. In this embodiment, the preservative system includes: 0.02% by weight to 0.2% by weight of a sorbic acid preservative selected from the group consisting of sorbic acid, esters thereof, salts thereof, and combinations thereof; 0 to 10.0% by weight of a preservative enhancing solubilizer; and 0.05% by weight to 0.2% by weight of a secondary preservative compound.

Certain embodiments of the present invention include a hydrophilic viscosity agent, such as those selected from cellulose ethers and carbomers. If used, the hydrophilic viscosity agent is preferably present in an amount of 0.2% by weight to 2.0% by weight. Other useful additives include, for example, a pH adjuster, an emulsifier, or combinations thereof.

Disclosed herein are the following methods :

A method of stabilizing a pharmaceutical formulation that includes: an immune response modifier (IRM) compound having a 2-aminopyridine moiety fused to a five-membered nitrogen-containing heterocyclic ring; and a preservative system that includes a sorbic acid preservative selected from the group consisting of sorbic acid, esters thereof, salts thereof, and combinations thereof. The method includes adding an antioxidant and optionally a chelating agent to the formulation.

A method for delivering an immune response modifier (IRM) to a dermal surface. The method includes selecting a formulation of the present invention and applying the selected formulation to the dermal and/or mucosal surface.

A method of treating a dermal associated condition (particularly, actinic keratosis). The method includes applying to a dermal surface of a patient in need thereof a pharmaceutical formulation of the present invention.

As used herein, a "sorbic acid preservative" means sorbic acid, esters of sorbic acid, salts of sorbic acid, or combinations thereof.

As used herein "remains substantially constant" means that the concentration of sorbic acid preservative in an IRM-containing formulation does not decrease by more than 15% of the initial concentration (i.e., its concentration when initially formulated) when stored for at least 6 months at 40°C and 75% relative humidity.

As used herein, "a" or "an" or "the" are used interchangeably with "at least one," to mean "one or more" of the listed element.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### DETAILED DESCRIPTION OF ILLUSTRATIVE EMBODIMENTS

The present invention provides topical formulations that include an immune response modifier containing a 2-aminopyridine fused to a 5-membered nitrogen-containing heterocyclic ring which is an imidazonaphthyridine amine, a preservative system that includes a sorbic acid preservative (i.e. sorbic acid, esters of sorbic acid, salts of sorbic acid or combinations thereof) and an antioxidant.

Surprisingly, such formulations are stabilized through the incorporation of the antioxidant, more preferably an antioxidant having hydrogen atom donating functionality. Additional stability, particularly of the antioxidant, can be obtained through the incorporation of a chelating agent.

Through the use of an antioxidant and an optional chelating agent, the concentration of the sorbic acid preservative in an IRM-containing formulation remains substantially constant relative to its initial concentration (i.e., its concentration when initially formulated) when stored for at least 6 months at 40°C and 75% relative humidity.

As used herein "remains substantially constant" means that the concentration of sorbic acid preservative in an IRM-containing formulation does not decrease by more than 15% of the initial concentration (i.e., its concentration when initially formulated) when stored for at least 6 months at 40°C and 75% relative humidity. Preferably, the concentration of the sorbic acid preservative in an IRM-containing formulation does not decrease by more than 10% of the initial concentration when stored for at least 6 months at 40°C and 75% relative humidity. More preferably, the concentration of the sorbic acid preservative in an IRM-containing formulation does not decrease by more than 5% of the initial concentration when stored for at least 6 months at 40°C and 75% relative humidity.

In certain embodiments, formulations described herein can be in the form of an oil-in-water emulsion such as a cream or a lotion. Such an emulsion can include an oil phase that includes one or more IRM compounds, a fatty acid in an amount sufficient to solubilize the IRM compound(s), a hydrophobic, aprotic component; and an aqueous phase that includes a preservative system, and a hydrophilic viscosity enhancing agent. Such components, as well as all others of the formulations described herein, are preferably pharmaceutically acceptable.

### Immune Response Modifiers

In one aspect, the present invention provides a formulation that includes an immune response modifier containing a 2-aminopyridine moiety fused to a five-membered nitrogen-containing heterocyclic ring, which is an imidazonaphthyridine amine

Immune response modifier compounds ("IRMs") include compounds that possess potent immunomodulating activity including but not limited to antiviral and antitumor activity. Certain IRMs modulate the production and secretion of cytokines. For example, certain IRM compounds induce the production and secretion of cytokines such as, e.g., Type I interferons, TNF-α, IL-1, IL-6, IL-8, IL-10, IL-12, MIP-1, and/or MCP-1. As another example, certain IRM compounds can inhibit production and secretion of certain T_{H}2 cytokines, such as IL-4 and IL-5. Additionally, some IRM compounds are said to suppress IL-1 and TNF (U.S. Patent No. 6,518,265).

IRM compounds to be used in the invention are imidazonaphthyridine amines. IRM compounds which are suitable for use are imidazoquinoline amines including, but not limited to, substituted imidazoquinoline amines such as, for example, amide substituted imidazoquinoline amines, sulfonamide substituted imidazoquinoline amines, urea substituted imidazoquinoline amines, aryl ether substituted imidazoquinoline amines, heterocyclic ether substituted imidazoquinoline amines, amido ether substituted imidazoquinoline amines, sulfonamido ether substituted imidazoquinoline amines, urea substituted imidazoquinoline ethers, thioether substituted imidazoquinoline amines, and 6-, 7-, 8-, or 9-aryl or heteroaryl substituted imidazoquinoline amines; tetrahydroimidazoquinoline amines including, but not limited to, amide substituted tetrahydroimidazoquinoline amines, sulfonamide substituted tetrahydroimidazoquinoline amines, urea substituted tetrahydroimidazoquinoline amines, aryl ether substituted tetrahydroimidazoquinoline amines, heterocyclic ether substituted tetrahydroimidazoquinoline amines, amido ether substituted tetrahydroimidazoquinoline amines, sulfonamido ether substituted tetrahydroimidazoquinoline amines, urea substituted tetrahydroimidazoquinoline ethers, and thioether substituted tetrahydroimidazoquinoline amines; imidazopyridine amines including, but not limited to, amide substituted imidazopyridine amines, sulfonamido substituted imidazopyridine amines, urea substituted imidazopyridine amines, aryl ether substituted imidazopyridine amines, heterocyclic ether substituted imidazopyridine amines, amido ether substituted imidazopyridine amines, sulfonamido ether substituted imidazopyridine amines, urea substituted imidazopyridine ethers, and thioether substituted imidazopyridine amines; 1,2-bridged imidazoquinoline amines; 6,7-fused cycloalkylimidazopyridine amines; tetrahydroimidazonaphthyridine amines; oxazoloquinoline amines; thiazoloquinoline amines; oxazolopyridine amines; thiazolopyridine amines; oxazolonaphthyridine amines; thiazolonaphthyridine amines; imidazoquinoline-1,4-diamines; and 1*H-*imidazo dimers fused to pyridine amines, quinoline amines, tetrahydroquinoline amines, naphthyridine amines, or tetrahydronaphthyridine amines.

These immune response modifier compounds, methods of making them, methods of using them and compositions containing them are disclosed in U.S. Patent Nos. 4,689,338; 4,929,624; 4,988,815; 5,037,986; 5,175,296; 5,238,944; 5,266,575; 5,268,376; 5,346,905; 5,352,784; 5,367,076; 5,389,640; 5,395,937; 5,446,153; 5,482,936; 5,693,811; 5,741,908; 5,756,747; 5,939,090; 6,039,969; 6,069,149; 6,083,505; 6,110,929; 6,194,425; 6,245,776; 6,331,539; 6,376,669; 6,451,810; 6,525,064; 6,541,485; 6,545,016; 6,545,017; 6,558,951; 6,573,273; 6,656,938; 6,660,735; 6,660,747; 6,664,260; 6,664,264; 6,664,265; 6,667,312; 6,670,372; 6,677,347; 6,677,348; 6,677,349; 6,683,088; 6,743,920; 6,756,382; 6,797,718; and 6,818,650; European Patent 0 394 026; U.S. Patent Publication Nos. 2002/0016332; 2002/0055517; 2002/0110840; 2003/0133913; 2003/0161797; 2003/0199538; 2004/0014779; 2004/0147543; 2004/0175336; 2004/0176367; 2004/0180919; 2004/0181130; 2004/0181211; and 2004/0192585.

As noted above, many of the IRM compounds useful in the present invention have demonstrated immunomodulating activity.
Disclosed herein are IRM compounds which can be chosen from 1H-imidazo[4,5-c]quinolin-4-amines defined by one of Formulas I-V below: 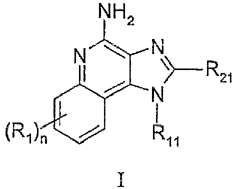 wherein
R₁₁ is selected from alkyl of one to ten carbon atoms, hydroxyalkyl of one to six carbon atoms, acyloxyalkyl wherein the acyloxy moiety is alkanoyloxy of two to four carbon atoms or benzoyloxy, and the alkyl moiety contains one to six carbon atoms, benzyl, (phenyl)ethyl and phenyl, said benzyl, (phenyl)ethyl or phenyl substituent being optionally substituted on the benzene ring by one or two moieties independently selected from alkyl of one to four carbon atoms, alkoxy of one to four carbon atoms and halogen, with the proviso that if said benzene ring is substituted by two of said moieties, then said moieties together contain no more than six carbon atoms;
R₂₁ is selected from hydrogen, alkyl of one to eight carbon atoms, benzyl, (phenyl)ethyl and phenyl, the benzyl, (phenyl)ethyl or phenyl substituent being optionally substituted on the benzene ring by one or two moieties independently selected from alkyl of one to four carbon atoms, alkoxy of one to four carbon atoms and halogen, with the proviso that when the benzene ring is substituted by two of said moieties, then the moieties together contain no more than six carbon atoms; and
each R₁ is independently selected from alkoxy of one to four carbon atoms, halogen, and alkyl of one to four carbon atoms, and n is an integer from 0 to 2, with the proviso that if n is 2, then said R₁ groups together contain no more than six carbon atoms; 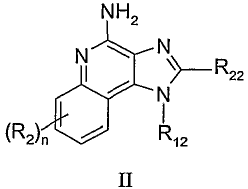
wherein
R₁₂ is selected from straight chain or branched chain alkenyl containing two to ten carbon atoms and substituted straight chain or branched chain alkenyl containing two to ten carbon atoms, wherein the substituent is selected from straight chain or branched chain alkyl containing one to four carbon atoms and cycloalkyl containing three to six carbon atoms; and cycloalkyl containing three to six carbon atoms substituted by straight chain or branched chain alkyl containing one to four carbon atoms; and
R₂₂ is selected from hydrogen, straight chain or branched chain alkyl containing one to eight carbon atoms, benzyl, (phenyl)ethyl and phenyl, the benzyl, (phenyl)ethyl or phenyl substituent being optionally substituted on the benzene ring by one or two moieties independently selected from straight chain or branched chain alkyl containing one to four carbon atoms, straight chain or branched chain alkoxy containing one to four carbon atoms, and halogen, with the proviso that when the benzene ring is substituted by two such moieties, then the moieties together contain no more than six carbon atoms; and
each R₂ is independently selected from straight chain or branched chain alkoxy containing one to four carbon atoms, halogen, and straight chain or branched chain alkyl containing one to four carbon atoms, and n is an integer from zero to 2, with the proviso that if n is 2, then said R₂ groups together contain no more than six carbon atoms; 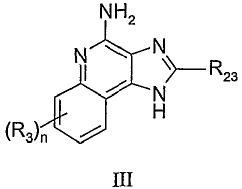
wherein
R₂₃ is selected from hydrogen, straight chain or branched chain alkyl of one to eight carbon atoms, benzyl, (phenyl)ethyl and phenyl, the benzyl, (phenyl)ethyl or phenyl substituent being optionally substituted on the benzene ring by one or two moieties independently selected from straight chain or branched chain alkyl of one to four carbon atoms, straight chain or branched chain alkoxy of one to four carbon atoms, and halogen, with the proviso that when the benzene ring is substituted by two such moieties, then the moieties together contain no more than six carbon atoms; and
each R₃ is independently selected from straight chain or branched chain alkoxy of one to four carbon atoms, halogen, and straight chain or branched chain alkyl of one to four carbon atoms, and n is an integer from zero to 2, with the proviso that if n is 2, then said R₃ groups together contain no more than six carbon atoms; 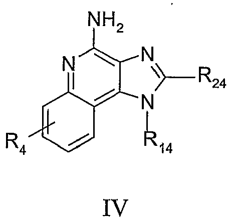
wherein
R₁₄ is -CHRₓR_{y} wherein R_{y} is hydrogen or a carbon-carbon bond, with the proviso that when R_{y} is hydrogen Rₓ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, or 2-, 3-, or 4-pyridyl, and with the further proviso that when R_{y} is a carbon-carbon bond R_{y} and Rₓ together form a tetrahydrofuranyl group optionally substituted with one or more substituents independently selected from hydroxy and hydroxyalkyl of one to four carbon atoms;
R₂₄ is selected from hydrogen, alkyl of one to four carbon atoms, phenyl, and substituted phenyl wherein the substituent is selected from alkyl of one to four carbon atoms, alkoxy of one to four carbon atoms, and halogen; and
R₄ is selected from hydrogen, straight chain or branched chain alkoxy containing one to four carbon atoms, halogen, and straight chain or branched chain alkyl containing one to four carbon atoms; 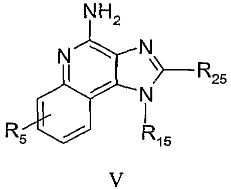
wherein
R₁₅ is selected from hydrogen; straight chain or branched chain alkyl containing one to ten carbon atoms and substituted straight chain or branched chain alkyl containing one to ten carbon atoms, wherein the substituent is selected from cycloalkyl containing three to six carbon atoms and cycloalkyl containing three to six carbon atoms substituted by straight chain or branched chain alkyl containing one to four carbon atoms; straight chain or branched chain alkenyl containing two to ten carbon atoms and substituted straight chain or branched chain alkenyl containing two to ten carbon atoms, wherein the substituent is selected from cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the alkyl moiety contains one to six carbon atoms; acyloxyalkyl wherein the acyloxy moiety is alkanoyloxy of two to four carbon atoms or benzoyloxy, and the alkyl moiety contains one to six carbon atoms; benzyl; (phenyl)ethyl; and phenyl; said benzyl, (phenyl)ethyl or phenyl substituent being optionally substituted on the benzene ring by one or two moieties independently selected from alkyl of one to four carbon atoms, alkoxy of one to four carbon atoms, and halogen, with the proviso that when said benzene ring is substituted by two of said moieties, then the moieties together contain no more than six carbon atoms;
R₂₅ is 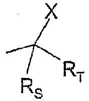
wherein
R_{S} and R_{T} are independently selected from hydrogen, alkyl of one to four carbon atoms, phenyl, and substituted phenyl wherein the substituent is selected from alkyl of one to four carbon atoms, alkoxy of one to four carbon atoms, and halogen;
X is selected from alkoxy containing one to four carbon atoms, alkoxyalkyl wherein the alkoxy moiety contains one to four carbon atoms and the alkyl moiety contains one to four carbon atoms, hydroxyalkyl of one to four carbon atoms, haloalkyl of one to four carbon atoms, alkylamido wherein the alkyl group contains one to four carbon atoms, amino, substituted amino wherein the substituent is alkyl or hydroxyalkyl of one to four carbon atoms, azido, chloro, hydroxy, 1-morpholino, 1-pyrrolidino, alkylthio of one to four carbon atoms; and
R₅ is selected from hydrogen, straight chain or branched chain alkoxy containing one to four carbon atoms, halogen, and straight chain or branched chain alkyl containing one to four carbon atoms;
and pharmaceutically acceptable salts of any of the foregoing.

Other disclosed IRM compounds can be chosen from 6,7 fused cycloalkylimidazopyridine amines defined by Formula VI below: 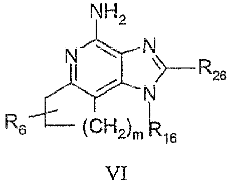 wherein
mis 1, 2, or 3;
R₁₆ is selected from hydrogen; cyclic alkyl of three, four, or five carbon atoms; straight chain or branched chain alkyl containing one to ten carbon atoms and substituted straight chain or branched chain alkyl containing one to ten carbon atoms, wherein the substituent is selected from cycloalkyl containing three to six carbon atoms and cycloalkyl containing three to six carbon atoms substituted by straight chain or branched chain alkyl containing one to four carbon atoms; fluoro- or chloroalkyl containing from one to ten carbon atoms and one or more fluorine or chlorine atoms; straight chain or branched chain alkenyl containing two to ten carbon atoms and substituted straight chain or branched chain alkenyl containing two to ten carbon atoms, wherein the substituent is selected from cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the alkyl moiety contains one to six carbon atoms; acyloxyalkyl wherein the acyloxy moiety is alkanoyloxy of two to four carbon atoms or benzoyloxy, and the alkyl moiety contains one to six carbon atoms, with the proviso that any such alkyl, substituted alkyl, alkenyl, substituted alkenyl, hydroxyalkyl, alkoxyalkyl, or acyloxyalkyl group does not have a fully carbon substituted carbon atom bonded directly to the nitrogen atom; benzyl; (phenyl)ethyl; and phenyl; said benzyl, (phenyl)ethyl or phenyl substituent being optionally substituted on the benzene ring by one or two moieties independently selected from alkyl of one to four carbon atoms, alkoxy of one to four carbon atoms, and halogen, with the proviso that when said benzene ring is substituted by two of said moieties, then the moieties together contain no more than six carbon atoms; and -CHRₓR_{y}
   wherein
R_{y} is hydrogen or a carbon-carbon bond, with the proviso that when R_{y} is hydrogen Rₓ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, 2-, 3-, or 4-pyridyl, and with the further proviso that when R_{y} is a carbon-carbon bond R_{y} and Rₓ together form a tetrahydrofuranyl group optionally substituted with one or more substituents independently selected from hydroxy and hydroxyalkyl of one to four carbon atoms,
R₂₆ is selected from hydrogen; straight chain or branched chain alkyl containing one to eight carbon atoms; straight chain or branched chain hydroxyalkyl containing one to six carbon atoms; morpholinoalkyl; benzyl; (phenyl)ethyl; and phenyl, the benzyl, (phenyl)ethyl, or phenyl substituent being optionally substituted on the benzene ring by a moiety selected from methyl, methoxy, and halogen; and -C(R_{S})(R_{T})(X) wherein R_{S} and R_{T} are independently selected from hydrogen, alkyl of one to four carbon atoms, phenyl, and substituted phenyl wherein the substituent is selected from alkyl of one to four carbon atoms, alkoxy of one to four carbon atoms, and halogen;
X is selected from alkoxy containing one to four carbon atoms, alkoxyalkyl wherein the alkoxy moiety contains one to four carbon atoms and the alkyl moiety contains one to four carbon atoms, haloalkyl of one to four carbon atoms, alkylamido wherein the alkyl group contains one to four carbon atoms, amino, substituted amino wherein the substituent is alkyl or hydroxyalkyl of one to four carbon atoms, azido, alkylthio of one to four carbon atoms, and morpholinoalkyl wherein the alkyl moiety contains one to four carbon atoms, and
R₆ is selected from hydrogen, fluoro, chloro, straight chain or branched chain alkyl containing one to four carbon atoms, and straight chain or branched chain fluoro-or chloroalkyl containing one to four carbon atoms and at least one fluorine or chlorine atom;
and pharmaceutically acceptable salts thereof.

Disclosed IRM compounds can also be chosen from imidazopyridine amines defined by Formula VII below: 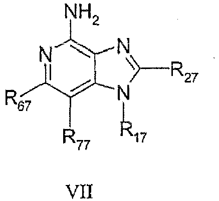 wherein
R₁₇ is selected from hydrogen; -CH₂R_{W} wherein R_{W} is selected from straight chain, branched chain, or cyclic alkyl containing one to ten carbon atoms, straight chain or branched chain alkenyl containing two to ten carbon atoms, straight chain or branched chain hydroxyalkyl, containing one to six carbon atoms, alkoxyalkyl wherein the alkoxy moiety contains one to four carbon atoms and the alkyl moiety contains one to six carbon atoms, and phenylethyl; and -CH=CR_{Z}R_{Z} wherein each R_{Z} is independently straight chain, branched chain, or cyclic alkyl of one to six carbon atoms;
R₂₇ is selected from hydrogen; straight chain or branched chain alkyl containing one to eight carbon atoms; straight chain or branched chain hydroxyalkyl containing one to six carbon atoms; alkoxyalkyl wherein the alkoxy moiety contains one to four carbon atoms and the alkyl moiety contains one to six carbon atoms; benzyl, (phenyl)ethyl and phenyl, the benzyl, (phenyl)ethyl and phenyl being optionally substituted on the benzene ring by a moiety selected from methyl, methoxy, and halogen; and morpholinoalkyl wherein the alkyl moiety contains one to four carbon atoms;
R₆₇ and R₇₇ are independently selected from hydrogen and alkyl of one to five carbon atoms, with the proviso that R₆₇ and R₇₇ taken together contain no more than six carbon atoms, and with the further proviso that when R₇₇ is hydrogen then R₆₇ is other than hydrogen and R₂₇ is other than hydrogen or morpholinoalkyl, and with the further proviso that when R₆₇ is hydrogen then R₇₇ and R₂₇ are other than hydrogen; and pharmaceutically acceptable salts thereof.

Other disclosed IRM compounds can be chosen from 1,2-bridged imidazoquinoline amines defined by Formula VIII below: 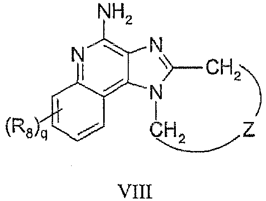 wherein
Z is selected from
-(CH₂)ₚ- wherein p is 1 to 4;
-(CH₂)ₐ-C(R_{D}R_{E})(CH₂)_{b}-, wherein a and b are integers and a+b is 0 to 3, R_{D} is hydrogen or alkyl of one to four carbon atoms, and R_{E} is selected from alkyl of one to four carbon atoms, hydroxy, -OR_{F} wherein R_{F} is alkyl of one to four carbon atoms, and -NR_{G}R'_{G} wherein R_{G} and R'_{G} are independently hydrogen or alkyl of one to four carbon atoms; and
-(CH₂)ₐ-(Y)-(CH₂)_{b}- wherein a and b are integers and a+b is 0 to 3, and Y is O, S, or -NR_{J}- wherein R_{J} is hydrogen or alkyl of one to four carbon atoms;
q is 0 or 1; and
R₈ is selected from alkyl of one to four carbon atoms, alkoxy of one to four carbon atoms, and halogen,
and pharmaceutically acceptable salts thereof.

Other disclosed IRM compounds can be chosen from thiazoloquinoline amines, oxazoloquinoline amines, thiazolopyridine amines, oxazolopyridine amines, thiazolonaphthyridine amines and oxazolonaphthyridine amines defined by Formula IX below: 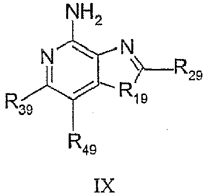 wherein:
R₁₉ is selected from oxygen, sulfur and selenium;
R₂₉ is selected from
   -hydrogen;
   -alkyl;
   -alkyl-OH;
   -haloalkyl;
   -alkenyl;
   -alkyl-X-alkyl;
   -alkyl-X-alkenyl;
   -alkenyl-X-alkyl;
   -alkenyl-X-alkenyl;
   -alkyl-N(R₅₉)₂;
   -alkyl-N₃;
   -alkyl-O-C(O)-N(R₅₉)₂;
   -heterocyclyl;
   -alkyl-X-heterocyclyl;
   -alkenyl-X-heterocyclyl;
   -aryl;
   -alkyl-X-aryl;
   -alkenyl-X-aryl;
   -heteroaryl;
   -alkyl-X-heteroaryl; and
   -alkenyl-X-heteroaryl;
R₃₉ and R₄₉ are each independently:
   -hydrogen;
   -X-alkyl;
   -halo;
   -haloalkyl;
   -N(R₅₉)₂;
   or when taken together, R₃₉ and R₄₉ form a fused
   aromatic, heteroaromatic, cycloalkyl or heterocyclic ring;
X is selected from -O- -S-, -NR₅₉-, -C(O)-, -C(O)O-, -OC(O)-, and a bond; and
each R₅₉ is independently H or C₁₋₈alkyl;
and pharmaceutically acceptable salts thereof.

In one embodiment, the IRM compound can be chosen from imidazonaphthyridine amines and imidazotetrahydronaphthyridine amines defined by Formulas X and XI below: 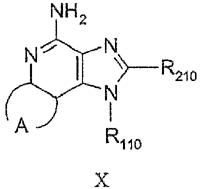 wherein
A is =N-CR=CR-CR=; =CR-N=CR-CR=; =CR-CR=N-CR=; or =CR-CR=CR-N=;
R₁₁₀ is selected from:
   - hydrogen;
   -C₁₋₂₀ alkyl or C₂₋₂₀ alkenyl that is unsubstituted or substituted by one or more substituents selected from:
      -aryl;
      -heteroaryl;
      -heterocyclyl;
      -O-C₁₋₂₀ alkyl;
      -O-(C₁₋₂₀ alkyl)₀₋₁-aryl;
      -O-(C₁₋₂₀ alkyl)₀₋₁-heteroaryl;
      -O-(C₁₋₂₀alkyl)₀₋₁-heterocyclyl;
      -CO-O-C₁₋₂₀ alkyl;
      -S(O)₀₋₂-C₁₋₂₀ alkyl;
      -S(O)₀₋₂-(C₁₋₂₀ alkyl)₀₋₁-aryl;
      -S(O)₀₋₂-(C₁₋₂₀ alkyl)₀₋₁-heteroaryl;
      -S(O)₀₋₂-(C₁₋₂₀ alkyl)₀₋₁-heterocyclyl;
      -N(R₃₁₀)₂;
      -N₃;
      oxo;
      -halogen;
      -NO₂;
      -OH; and
      -SH; and
   -C₁₋₂₀ alkyl-NR₃₁₀-Q-X-R₄₁₀ or -C₂₋₂₀ alkenyl-NR₃₁₀-Q-X-R₄₁₀ wherein Q is - CO- or -SO₂-; X is a bond, -O- or -NR₃₁₀- and R₄₁₀ is aryl; heteroaryl; heterocyclyl; or - C₁₋₂₀ alkyl or C₂₋₂₀ alkenyl that is unsubstituted or substituted by one or more substituents selected from:
      -aryl;
      -heteroaryl;
      -heterocyclyl;
      -O-C₁₋₂₀ alkyl;
      -O-(C₁₋₂₀ alkyl)₀₋₁-aryl;
      -O-(C₁₋₂₀ alkyl)₀₋₁-heteroaryl;
      -O-(C₁₋₂₀ alkyl)₀₋₁-heterocyclyl;
      -CO-O-C₁₋₂₀ alkyl;
      -S(O)₀₋₂ -C₁₋₂₀ alkyl;
      -S(O)₀₋₂-(C₁₋₂₀ alkyl)₀₋₁-aryl;
      -S(O)₀₋₂-(C₁₋₂₀ alkyl)₀₋₁-heteroaryl;
      -S(O)₀₋₂-(C₁₋₂₀ alkyl)₀₋₁-heterocyclyl;
      -N(R₃₁₀)₂;
      -NR₃₁₀-CO-O-C₁₋₂₀ alkyl;
      -N₃;
      oxo;
      -halogen;
      -NO₂;
      -OH; and
      -SH; or R₄₁₀ is 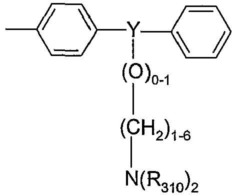
      wherein Y is N- or -CR-;
R₂₁₀ is selected from:
   -hydrogen;
   -C₁₋₁₀ alkyl;
   -C₂₋₁₀ alkenyl;
   -aryl;
   -C₁₋₁₀ alkyl-O-C₁₋₁₀ alkyl;
   -C₁₋₁₀ alkyl-O-C₂₋₁₀ alkenyl; and
   -C₁₋₁₀ alkyl or C₂₋₁₀ alkenyl substituted by one or more substituents selected from:
      -OH;
      -halogen;
      -N(R₃₁₀)₂;
      -CO-N(R₃₁₀)₂;
      -CO-C₁₋₁₀ alkyl;
      -N₃;
      -aryl;
      -heteroaryl;
      -heterocyclyl;
      -CO-aryl; and
      -CO-heteroaryl;
   each R₃₁₀ is independently selected from hydrogen and C₁₋₁₀ alkyl; and
   each R is independently selected from hydrogen, C₁₋₁₀ alkyl, C₁₋₁₀ alkoxy, halogen and trifluoromethyl; 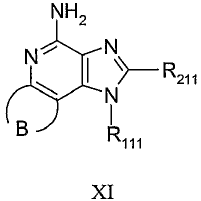
wherein
B is -NR-C(R)₂-C(R)₂-C(R)₂-; -C(R)₂-NR-C(R)₂-C(R)₂-; -C(R)₂-C(R)₂-NR-C(R)₂- or -C(R)₂-C(R)₂-C(R)₂-NR-;
R₁₁₁ is selected from:
- hydrogen;
-C₁₋₂₀ alkyl or C₂₋₂₀ alkenyl that is unsubstituted or substituted by one or more substituents selected from:
   -aryl;
   -heteroaryl;
   -heterocyclyl;
   -O-C₁₋₂₀alkyl;
   -O-(C₁₋₂₀alkyl)₀₋₁-aryl;
   -O-(C₁₋₂₀ alkyl)₀₋₁-heteroaryl;
   -O-(C₁₋₂₀ alkyl)₀₋₁-heterocyclyl;
   -CO-O-C₁₋₂₀ alkyl;
   -S(O)₀₋₂-C₁₋₂₀ alkyl;
   -S(O)₀₋₂-(C₁₋₂₀ alkyl)₀₋₁-aryl;
   -S(O)₀₋₂-(C₁₋₂₀ alkyl)₀₋₁-heteroaryl;
   -S(O)₀₋₂-(C₁₋₂₀ alkyl)₀₋₁-heterocyclyl;
   -N(R₃₁₁)₂;
   -N₃;
   oxo;
   -halogen;
   -NO₂;
   -OH; and
   -SH; and
-C₁₋₂₀ alkyl-NR₃₁₁-Q-X-R₄₁₁ or -C₂₋₂₀ alkenyl-NR₃₁₁-Q-X-R₄₁₁ wherein Q is - CO- or -SO₂-; X is a bond, -O- or -NR₃₁₁- and R₄₁₁ is aryl; heteroaryl; heterocyclyl; or - C₁₋₂₀ alkyl or C₂₋₂₀ alkenyl that is unsubstituted or substituted by one or more substituents selected from:
   -aryl;
   -heteroaryl;
   -heterocyclyl;
   -O-C₁₋₂₀ alkyl;
   -O-(C₁₋₂₀ alkyl)₀₋₁-aryl;
   -O-(C₁₋₂₀ alkyl)₀₋₁-heteroaryl;
   -O-(C₁₋₂₀ alkyl)₀₋₁-heterocyclyl;
   -CO-O-C₁₋₂₀ alkyl;
   -S(O)₀₋₂-C₁₋₂₀ alkyl;
   -S(O)₀₋₂-(C₁₋₂₀ alkyl)₀₋₁-aryl;
   -S(O)₀₋₂-(C₁₋₂₀ alkyl)₀₋₁-heteroaryl;
   -S(O)₀₋₂-(C₁₋₂₀ alkyl)₀₋₁-heterocyclyl;
   -N(R₃₁₁)₂;
   -NR₃₁₁-CO-O-C₁₋₂₀ alkyl;
   -N₃;
   oxo;
   -halogen;
   -NO₂;
   -OH; and
   -SH; or R₄₁₁ is 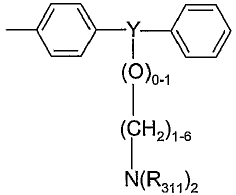 wherein Y is N- or -CR-;
R₂₁₁ is selected from:
   -hydrogen;
   -C₁₋₁₀ alkyl;
   -C₂₋₁₀ alkenyl;
   -aryl;
   -C₁₋₁₀ alkyl -O-C₁₋₁₀-alkyl;
   -C₁₋₁₀ alkyl-O-C₂₋₁₀ alkenyl; and
   -C₁₋₁₀ alkyl or C₂₋₁₀ alkenyl substituted by one or more substituents selected from:
      -OH;
      -halogen;
      -N(R₃₁₁)₂;
      -CO-N(R₃₁₁)₂;
      -CO-C₁₋₁₀ alkyl;
      -N₃;
      -aryl;
      -heteroaryl;
      -heterocyclyl;
      -CO-aryl; and
      -CO-heteroaryl;
   each R₃₁₁ is independently selected from hydrogen and C₁₋₁₀ alkyl; and
   each R is independently selected from hydrogen, C₁₋₁₀ alkyl C₁₋₁₀ alkoxy, halogen, and trifluoromethyl;
   and pharmaceutically acceptable salts thereof.

Other disclosed IRM compounds can be chosen from 1*H-*imdazo[4,5-*c*]quinolin-4-amines and tetrahydro-1*H*-imidazo[4,5-*c*]quinolin-4-amines defined by Formulas XII, XIII and XIV below: 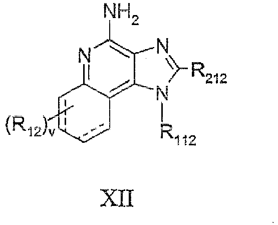 wherein
R₁₁₂ is -alkyl-NR₃₁₂-CO-R₄₁₂ or -alkenyl-NR₃₁₂-CO- R₄₁₂ wherein R₄₁₂ is aryl, heteroaryl, alkyl or alkenyl, each of which may be unsubstituted or substituted by one or more substituents selected from:
   -alkyl;
   -alkenyl;
   -alkynyl;
   -(alkyl)₀₋₁-aryl;
   -(alkyl)₀₋₁-(substituted aryl);
   -(alkyl)₀₋₁-heteroaryl;
   -(alkyl)₀₋₁-(substituted heteroaryl);
   -O-alkyl;
   -O-(alkyl)₀₋₁-aryl;
   -O-(alkyl)₀₋₁-(substituted aryl);
   -O-(alkyl)₀₋₁-heteroaryl;
   -O-(alkyl)₀₋₁-(substituted heteroaryl);
   -CO-aryl;
   -CO-(substituted aryl);
   -CO-heteroaryl;
   -CO-(substituted heteroaryl);
   -COOH;
   -CO-O-alkyl;
   -CO-alkyl;
   -S(O)₀₋₂ -alkyl;
   -S(O)₀₋₂-(alkyl)₀₋₁-aryl;
   -S(O)₀₋₂-(alkyl)₀₋₁-(substituted aryl);
   -S(O)₀₋₂-(alkyl)₀₋₁-heteroaryl;
   -S(O)₀₋₂-(alkyl)₀₋₁-(substituted heteroaryl);
   -P(O)(OR₃₁₂)₂;
   -NR₃₁₂-CO-O-alkyl;
   -N₃;
   -halogen;
   -NO₂;
   -CN;
   -haloalkyl;
   -O-haloalkyl;
   -CO-haloalkyl;
   -OH;
   -SH; and in the case that R₄₁₂ is alkyl, alkenyl, or heterocyclyl, oxo; or R₄₁₂ is 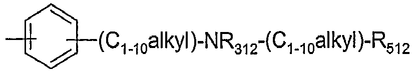 wherein R₅₁₂ is an aryl, (substituted aryl), heteroaryl, (substituted heteroaryl), heterocyclyl or (substituted heterocyclyl) group;
R₂₁₂ is selected from:
   -hydrogen;
   -alkyl;
   -alkenyl;
   -aryl;
   -(substituted aryl);
   -heteroaryl;
   -(substituted heteroaryl);
   -heterocyclyl;
   -(substituted heterocyclyl);
   -alkyl-O-alkyl;
   -alkyl-O-alkenyl; and
   -alkyl or alkenyl substituted by one or more substituents selected from:
      -OH;
      -halogen;
      -N(R₃₁₂)₂;
      -CO-N(R₃₁₂)₂;
      -CO-C₁₋₁₀ alkyl;
      -CO-O-C₁₋₁₀ alkyl;
      -N₃;
      -aryl;
      -(substituted aryl);
      -heteroaryl;
      -(substituted heteroaryl);
      -heterocyclyl;
      -(substituted heterocyclyl);
      -CO-aryl; and
      -CO-heteroaryl;
each R₃₁₂ is independently selected from hydrogen; C₁₋₁₀ alkyl-heteroaryl; C₁₋₁₀ alkyl-(substituted heteroaryl); C₁₋₁₀ alkyl-aryl; C₁₋₁₀ alkyl-(substituted aryl) and C₁₋₁₀ alkyl;
v is 0 to 4;
and each R₁₂ present is independently selected from C₁₋₁₀ alkyl, C₁₋₁₀ alkoxy, halogen, and trifluoromethyl; 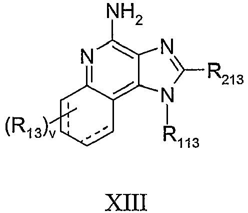
wherein
R₁₁₃ is -alkyl-NR₃₁₃- SO₂ -X-R₄₁₃ or -alkenyl-NR₃₁₃- SO₂ -X-R₄₁₃ ;
X is a bond or -NR₅₁₃-;
R₄₁₃ is aryl, heteroaryl, heterocyclyl, alkyl or alkenyl, each of which may be unsubstituted or substituted by one or more substituents selected from:
   -alkyl;
   -alkenyl;
   -aryl;
   -heteroaryl;
   -heterocyclyl;
   -substituted cycloalkyl;
   -substituted aryl;
   -substituted heteroaryl;
   -substituted heterocyclyl;
   -O-alkyl;
   -O-(alkyl)₀₋₁-aryl;
   -O-(alkyl)₀₋₁-substituted aryl;
   -O-(alkyl)₀₋₁-heteroaryl;
   -O-(alkyl)₀₋₁-substituted heteroaryl;
   -O-(alkyl)₀₋₁-heterocyclyl;
   -O-(alkyl)₀₋₁-substituted heterocyclyl;
   -COOH;
   -CO-O-alkyl;
   -CO-alkyl;
   -S(O)₀₋₂-alkyl;
   -S(O)₀₋₂-(alkyl)₀₋₁-aryl;
   -S(O)₀₋₂ -(alkyl)₀₋₁-substituted aryl;
   -S(O)₀₋₂-(alkyl)₀₋₁-heteroaryl;
   -S(O)₀₋₂-(alkyl)₀₋₁-substituted heteroaryl;
   -S(O)₀₋₂ -(alkyl)₀₋₁-heterocyclyl;
   -S(O)₀₋₂-(alkyl)₀₋₁-substituted heterocyclyl;
   -(alkyl)₀₋₁-NR₃₁₃R₃₁₃;
   -(alkyl)₀₋₁-NR₃₁₃-CO-O-alkyl;
   -(alkyl)₀₋₁-NR₃₁₃-CO-alkyl;
   -(alkyl)₀₋₁-NR₃₁₃-CO-aryl;
   -(alkyl)₀₋₁-NR₃₁₃-CO-substituted aryl;
   -(alkyl)₀₋₁-NR₃₁₃-CO-heteroaryl;
   -(alkyl)₀₋₁-NR₃₁₃-CO-substituted heteroaryl;
   -N₃;
   -halogen;
   -haloalkyl;
   -haloalkoxy;
   -CO-haloalkyl;
   -CO-haloalkoxy;
   -NO₂;
   -CN;
   -OH;
   -SH; and in the case that R₄₁₃ is alkyl, alkenyl, or heterocyclyl, oxo;
R₂₁₃ is selected from:
   -hydrogen;
   -alkyl;
   -alkenyl;
   -aryl;
   -substituted aryl;
   -heteroaryl;
   -substituted heteroaryl;
   - alkyl-O-alkyl;
   - alkyl-O- alkenyl; and
   - alkyl or alkenyl substituted by one or more substituents selected from:
      -OH;
      -halogen;
      -N(R₃₁₃)₂;
      -CO-N(R₃₁₃)₂;
      -CO-C₁₋₁₀ alkyl;
      -CO-O-C₁₋₁₀ alkyl;
      -N₃;
      -aryl;
      -substituted aryl;
      -heteroaryl;
      -substituted heteroaryl;
      -heterocyclyl;
      -substituted heterocyclyl;
      -CO-aryl;
      -CO-(substituted aryl);
      -CO-heteroaryl; and
      -CO-(substituted heteroaryl);
each R₃₁₃ is independently selected from hydrogen and C₁₋₁₀ alkyl; or when X is a bond R₃₁₃ and R₄₁₃ can join to form a 3 to 7 membered heterocyclic or substituted heterocyclic ring;
R₅₁₃ is selected from hydrogen and C₁₋₁₀ alkyl, or R₄₁₃ and R₅₁₃ can combine to form a 3 to 7 membered heterocyclic or substituted heterocyclic ring;
v is 0 to 4;
and each R₁₃ present is independently selected from C₁₋₁₀ alkyl, C₁₋₁₀ alkoxy, halogen, and trifluoromethyl; 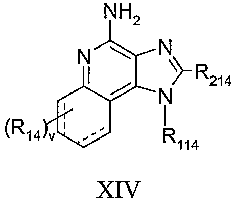
wherein
R₁₁₄ is -alkyl-NR₃₁₄-CY-NR₅₁₄-X-R₄₁₄ or
-alkenyl-NR₃₁₄-CY-NR₅₁₄-X-R₄₁₄
wherein
Y is =O or =S;
X is a bond, -CO- or -SO₂-;
R₄₁₄ is aryl, heteroaryl, heterocyclyl, alkyl or alkenyl, each of which maybe unsubstituted or substituted by one or more substituents selected from:
   -alkyl;
   -alkenyl;
   -aryl;
   -heteroaryl;
   -heterocyclyl;
   -substituted aryl;
   -substituted heteroaryl;
   -substituted heterocyclyl;
   -O-alkyl;
   -O-(alkyl)₀₋₁-aryl;
   -O-(alkyl)₀₋₁-substituted aryl;
   -O-(alkyl)₀₋₁-heteroaryl;
   -O-(alkyl)₀₋₁-substituted heteroaryl;
   -O-(alkyl)₀₋₁-heterocyclyl;
   -O-(alkyl)₀₋₁-substituted heterocyclyl;
   -COOH;
   -CO-O-alkyl;
   -CO-alkyl;
   -S(O)₀₋₂-alkyl;
   -S(O)₀₋₂-(alkyl)₀₋₁-aryl;
   -S(O)₀₋₂-(alkyl)₀₋₁-substituted aryl;
   -S(O)₀₋₂-(alkyl)₀₋₁-heteroaryl;
   -S(O)₀₋₂-(alkyl)₀₋₁-substituted heteroaryl;
   -S(O)₀₋₂-(alkyl)₀₋₁-heterocyclyl;
   -S(O)₀₋₂-(alkyl)₀₋₁-substituted heterocyclyl;
   -(alkyl)₀₋₁-NR₃₁₄R₃₁₄;
   -(alkyl)₀₋₁-NR₃₁₄-CO-O-alkyl;
   -(alkyl)₀₋₁-NR₃₁₄-CO-alkyl;
   -(alkyl)₀₋₁-NR₃₁₄-CO-aryl;
   -(alkyl)₀₋₁-NR₃₁₄-CO-substituted aryl;
   -(alkyl)₀₋₁-NR₃₁₄-CO-heteroaryl;
   -(alkyl)₀₋₁-NR₃₁₄-CO-substituted heteroaryl;
   -N₃;
   -halogen;
   -haloalkyl;
   -haloalkoxy;
   -CO-haloalkoxy;
   -NO₂;
   -CN;
   -OH;
   -SH; and, in the case that R₄₁₄ is alkyl, alkenyl or heterocyclyl, oxo;
   with the proviso that when X is a bond R₄₁₄ can additionally be hydrogen;
R₂₁₄ is selected from:
   -hydrogen;
   -alkyl;
   -alkenyl;
   -aryl;
   -substituted aryl;
   -heteroaryl;
   -substituted heteroaryl;
   - alkyl-O-alkyl;
   -alkyl-O- alkenyl; and
   - alkyl or alkenyl substituted by one or more substituents selected from:
      -OH;
      -halogen;
      -N(R₃₁₄)₂;
      -CO-N(R₃₁₄)₂;
      -CO-C₁₋₁₀ alkyl;
      -CO-O-C₁₋₁₀ alkyl;
      -N₃;
      -aryl;
      -substituted aryl;
      -heteroaryl;
      -substituted heteroaryl;
      -heterocyclyl;
      -substituted heterocyclyl;
      -CO-aryl;
      -CO-(substituted aryl);
      -CO-heteroaryl; and
      -CO-(substituted heteroaryl);
each R₃₁₄ is independently selected from hydrogen and C₁₋₁₀ alkyl;
R₅₁₄ is selected from hydrogen and C₁₋₁₀ alkyl, or R₄₁₄ and R₅₁₄ can combine to form a 3 to 7 membered heterocyclic or substituted heterocyclic ring;
v is 0 to 4;
and each R₁₄ present is independently selected from C₁₋₁₀ alkyl, C₁₋₁₀ alkoxy, halogen, and trifluoromethyl;
and pharmaceutically acceptable salts thereof.

Other disclosed IRM compounds can be chosen from 1*H-*imidazo[4,5-*c*]quinolin-4-amines and tetrahydro- 1*H*-imidazo[4,5-*c*]quinolin-4-amines defined by Formulas XV, XVI, XVII, XVIII, XIX, XX, XXI, XXII, XXIII, XXIV, XXV, and XXVI below: 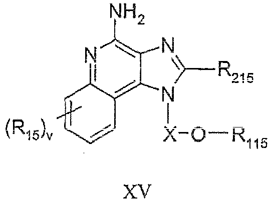
wherein: X is -CHR₅₁₅-, -CHR₅₁₅-alkyl-, or -CHR₅₁₅-alkenyl-;
R₁₁₅ is selected from:
   -R₄₁₅-CR₃₁₅-Z-R₆₁₅-alkyl;
   -R₄₁₅-CR₃₁₅-Z-R₆₁₅-alkenyl;
   -R₄₁₅-CR₃₁₅-Z-R₆₁₅-aryl;
   -R₄₁₅-CR₃₁₅-Z-R₆₁₅-heteroaryl;
   -R₄₁₅-CR₃₁₅-Z-R₆₁₅-heterocyclyl;
   -R₄₁₅-CR₃₁₅-Z-H;
   -R₄₁₅-NR₇₁₅-CR₃₁₅-R₆₁₅-alkyl;
   -R₄₁₅-NR₇₁₅-CR₃₁₅-R₆₁₅-alkenyl;
   -R₄₁₅-NR₇₁₅-CR₃₁₅-R₆₁₅-aryl;
   -R₄₁₅-NR₇₁₅-CR₃₁₅-R₆₁₅-heteroaryl;
   -R₄₁₅-NR₇₁₅-CR₃₁₅-R₆₁₅-heterocyclyl; and
   -R₄₁₅-NR₇₁₅-CR₃₁₅-R₈₁₅;
Z is -NR₅₁₅-, -O-, or -S-;
R₂₁₅ is selected from:
   -hydrogen;
   -alkyl;
   -alkenyl;
   -aryl;
   -heteroaryl;
   -heterocyclyl;
   -alkyl-Y-alkyl;
   -alkyl-Y-alkenyl;
   -alkyl-Y-aryl; and
   - alkyl or alkenyl substituted by one or more substituents selected from:
      -OH;
      -halogen;
      -N(R₅₁₅)₂;
      -CO-N(R₅₁₅)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
R₃₁₅ is =O or =S;
R₄₁₅ is alkyl or alkenyl, which may be interrupted by one or more -O- groups;
each R₅₁₅ is independently H or C₁₋₁₀ alkyl;
R₆₁₅ is a bond, alkyl, or alkenyl, which may be interrupted by one or more -O- groups;
R₇₁₅ is H, C₁₋₁₀ alkyl, or arylalkyl; or R₄₁₅ and R₇₁₅ can join together to form a ring;
R₈₁₅ is H or C₁₋₁₀ alkyl; or R₇₁₅ and R₈₁₅ can join together to form a ring; Y is -O- or -S(O)₀₋₂-;
v is 0 to 4; and
each R₁₅ present is independently selected from C₁₋₁₀ alkyl, C₁₋₁₀ alkoxy, hydroxy, halogen, and trifluoromethyl;

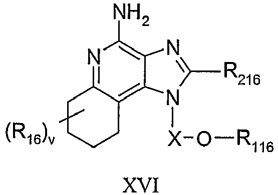

wherein: X is -CHR₅₁₆-, -CHR₅₁₆-alkyl-, or -CHR₅₁₆-alkenyl-;
R₁₁₆ is selected from:
   -R₄₁₆-CR₃₁₆-Z-R₆₁₆-alkyl;
   -R₄₁₆-CR₃₁₆-Z-R₆₁₆-alkenyl;
   -R₄₁₆-CR₃₁₆-Z-R₆₁₆-aryl;
   -R₄₁₆-CR₃₁₆-Z-R₆₁₆-heteroaryl;
   -R₄₁₆-CR₃₁₆-Z-R₆₁₆ heterocyclyl;
   -R₄₁₆-CR₃₁₆-Z-H,
   -R₄₁₆-NR₇₁₆-CR₃₁₆-R₆₁₆-alkyl;
   -R₄₁₆-NR₇₁₆-CR₃₁₆-R₆₁₆-alkenyl;
   -R₄₁₆-NR₇₁₆-CR₃₁₆-R₆₁₆-aryl;
   -R₄₁₆-NR₇₁₆-CR₃₁₆-R₆₁₆-heteroaryl;
   -R₄₁₆-NR₇₁₆-CR₃₁₆-R₆₁₆-heterocyclyl; and
   -R₄₁₆-NR₇₁₆-CR₃₁₆-R₈₁₆;
Z is -NR₅₁₆-, -O-, or -S-;
R₂₁₆ is selected from:
   - hydrogen;
   - alkyl;
   - alkenyl;
   - aryl;
   - heteroaryl;
   - heterocyclyl;
   - alkyl-Y-alkyl;
   - alkyl-Y- alkenyl;
   - alkyl-Y-aryl; and
   - alkyl or alkenyl substituted by one or more substituents selected from:
      -OH;
      -halogen;
      -N(R₅₁₆)₂;
      -CO-N(R₅₁₆)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
R₃₁₆ is =O or =S;
R₄₁₆ is alkyl or alkenyl, which may be interrupted by one or more -O- groups;
each R₅₁₆ is independently H or C₁₋₁₀ alkyl;
R₆₁₆ is a bond, alkyl, or alkenyl, which may be interrupted by one or more -O- groups;
R₇₁₆ is H, C₁₋₁₀ alkyl, arylalkyl; or R₄₁₆ and R₇₁₆ can join together to form a ring;
R₈₁₆ is H or C₁₋₁₀ alkyl; or R₇₁₆ and R₈₁₆ can join together to form a ring; Y is -O- or -S(O)₀₋₂-;
v is 0 to 4; and
each R₁₆ present is independently selected from C₁₋₁₀ alkyl, C₁₋₁₀ alkoxy, hydroxy, halogen, and trifluoromethyl;

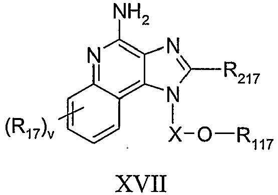

wherein: X is -CHR₃₁₇-, -CHR₃₁₇-alkyl-, or -CHR₃₁₇-alkenyl-;
R₁₁₇ is selected from:
   -alkenyl;
   -aryl; and
   -R₄₁₇-aryl;
R₂₁₇ is selected from:
   -hydrogen;
   -alkyl;
   -alkenyl;
   -aryl;
   -heteroaryl;
   -heterocyclyl;
   -alkyl-Y-alkyl;
   -alkyl-Y-alkenyl;
   -alkyl-Y-aryl; and
   - alkyl or alkenyl substituted by one or more substituents selected from:
      -OH;
      -halogen;
      -N(R₃₁₇)₂;
      -CO-N(R₃₁₇)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
R₄₁₇ is alkyl or alkenyl, which may be interrupted by one or more -O- groups;
each R₃₁₇ is independently H or C₁₋₁ alkyl;
each Y is independently -O- or -S(O)₀₋₂-;
v is 0 to 4; and
each R₁₇ present is independently selected from C₁₋₁₀ alkyl, C₁₋₁₀ alkoxy, hydroxy, halogen, and trifluoromethyl;

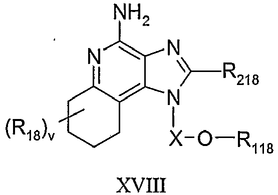

wherein: X is -CHR₃₁₈-, -CHR₃₁₈-alkyl-, or -CHR₃₁₈-alkenyl-;
R₁₁₈ is selected from:
   -aryl;
   -alkenyl; and
   -R₄₁₈-aryl;
R₂₁₈ is selected from:
   -hydrogen;
   -alkyl;
   -alkenyl;
   -aryl;
   -heteroaryl;
   -heterocyclyl;
   -alkyl-Y-alkyl;
   -alkyl-Y-aryl;
   - alkyl-Y-alkenyl; and
   - alkyl or alkenyl substituted by one or more substituents selected from:
      -OH;
      -halogen;
      -N(R₃₁₈)₂;
      -CO-N(R₃₁₈)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
R₄₁₈ is alkyl or alkenyl, which may be interrupted by one or more -O- groups;
each R₃₁₈ is independently H or C₁₋₁₀ alkyl;
each Y is independently -O- or -S(O)₀₋₂-;
v is 0 to 4; and
each R₁₈ present is independently selected C₁₋₁₀ alkyl, C₁₋₁₀ alkoxy, hydroxy, halogen, and trifluoromethyl;

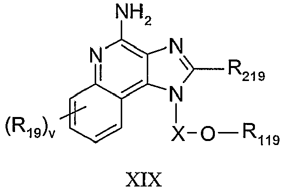

wherein: X is -CHR₃₁₉-, -CHR₃₁₉-alkyl-, or -CHR₃₁₉-alkenyl-;
R₁₁₉ is selected from:
   -heteroaryl;
   -heterocyclyl;
   -R₄₁₉- heteroaryl; and
   -R₄₁₉-heterocyclyl;
R₂₁₉ is selected from:
   -hydrogen;
   -alkyl;
   -alkenyl;
   -aryl;
   -heteroaryl;
   -heterocyclyl;
   -alkyl-Y-alkyl;
   -alkyl-Y-alkenyl;
   -alkyl-Y-aryl; and
   - alkyl or alkenyl substituted by one or more substituents selected from:
      -OH;
      -halogen;
      -N(R₃₁₉)₂;
      -CO-N(R₃₁₉)₂,
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
R₄₁₉ is alkyl or alkenyl, which may be interrupted by one or more -O- groups;
each R₃₁₉ is independently H or C₁₋₁₀ alkyl;
each Y is independently -O- or -S(O)₀₋₂-;
v is 0 to 4; and
each R₁₉ present is independently selected from C₁₋₁₀ alkyl, C₁₋₁₀ alkoxy, hydroxy, halogen, and trifluoromethyl;

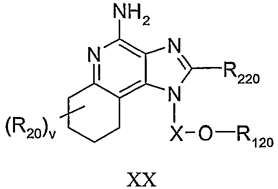

wherein: X is -CHR₃₂₀-, -CHR₃₂₀-alkyl-, or -CHR₃₂₀-alkenyl-;
R₁₂₀ is selected from:
   -heteroaryl;
   -heterocyclyl;
   -R₄₂₀- heteroaryl; and
   -R₄₂₀-heterocyclyl;
R₂₂₀ is selected from:
   -hydrogen;
   -alkyl;
   -alkenyl;
   -aryl;
   -heteroaryl;
   -heterocyclyl;
   -alkyl-Y-alkyl;
   -alkyl-Y- alkenyl;
   -alkyl-Y-aryl; and
   - alkyl or alkenyl substituted by one or more substituents selected from:
      -OH;
      -halogen;
      -N(R₃₂₀)₂;
      -CO-N(R₃₂₀)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
R₄₂₀ is alkyl or alkenyl, which may be interrupted by one or more -O- groups;
each R₃₂₀ is independently H or C₁₋₁₀ alkyl;
each Y is independently -O- or -S(O)₀₋₂-;
v is 0 to 4; and
each R₂₀ present is independently selected from C₁₋₁₀ alkyl, C₁₋₁₀ alkoxy, hydroxy, halogen, and trifluoromethyl;

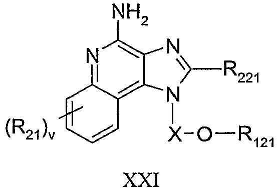

wherein: X is -CHR₅₂₁-, -CHR₅₂₁-alkyl-, or -CHR₅₂₁-alkenyl-;
R₁₂₁ is selected from:
   -R₄₂₁-NR₃₂₁-SO₂-R₆₂₁-alkyl;
   -R₄₂₁-NR₃₂₁-SO₂-R₆₂₁-alkenyl;
   -R₄₂₁-NR₃₂₁-SO₂-R₆₂₁-aryl;
   -R₄₂₁-NR₃₂₁-SO₂-R₆₂₁-heteroaryl;
   -R₄₂₁-NR₃₂₁-SO₂-R₆₂₁-heterocyclyl;
   -R₄₂₁-NR₃₂₁-SO₂-R₇₂₁;
   -R₄₂₁-NR₃₂₁-SO₂-NR₅₂₁-R₆₂₁-alkyl;
   -R₄₂₁-NR₃₂₁-SO₂-NR₅₂₁-R₆₂₁-alkenyl;
   -R₄₂₁-NK-₃₂₁-SO₂-NR₅₂₁-R-₆₂₁-aryl;
   -R₄₂₁-NR₃₂₁-SO₂-NR₅₂₁-R₆₂₁-heteroaryl;
   -R₄₂₁-NR₃₂₁-SO₂-NR₅₂₁-R₆₂₁-heterocyclyl; and
   -R₄₂₁-NR₃₂₁-SO₂-NH₂;
R₂₂₁ is selected from:
   -hydrogen;
   -alkyl;
   -alkenyl;
   -aryl;
   -heteroaryl;
   -heterocyclyl;
   -alkyl-Y-alkyl;
   -alkyl-Y- alkenyl;
   -alkyl-Y-aryl; and
   - alkyl or alkenyl substituted by one or more substituents selected from:
      -OH;
      -halogen;
      -N(R₅₂₁)₂;
      -CO-N(R₅₂₁)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
Y is -O- or -S(O)₀₋₂-;
R₃₂₁ is H, C₁₋₁₀ alkyl, or arylalkyl;
each R₄₂₁ is independently alkyl or alkenyl, which may be interrupted by one or more -O- groups; or R₃₂₁ and R₄₂₁ can join together to form a ring;
each R₅₂₁ is independently H, C₁₋₁₀ alkyl, or C₂₋₁₀ alkenyl;
R₆₂₁ is a bond, alkyl, or alkenyl, which may be interrupted by one or more -O- groups;
R₇₂₁ is C₁₋₁₀ alkyl; or R₃₂₁ and R₇₂₁ can join together to form a ring;
v is 0 to 4; and
each R₂₁ present is independently selected from C₁₋₁₀ alkyl, C₁₋₁₀ alkoxy, hydroxy, halogen, and trifluoromethyl;

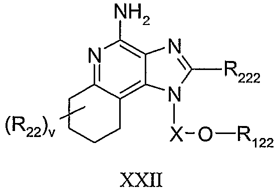

wherein: X is -CHR₅₂₂-, -CHR₅₂₂-alkyl-, or -CHR₅₂₂-alkenyl-;
R₁₂₂ is selected from:
   -R₄₂₂-NR₃₂₂-SO₂-R₆₂₂-alkyl;
   -R₄₂₂-NR₃₂₂-SO₂-R₆₂₂-alkenyl;
   -R₄₂₂-NR₃₂₂-SO₂-R₆₂₂-aryl;
   -R₄₂₂-NR₃₂₂-SO₂-R₆₂₂-heteroaryl;
   -R₄₂₂-NR₃₂₂-SO₂-R₆₂₂-heterocyclyl;
   -R₄₂₂-NR₃₂₂-SO₂-R₇₂₂;
   -R₄₂₂-NR₃₂₂-SO₂-NR₅₂₂-R₆₂₂-alkyl;
   -R₄₂₂-NR₃₂₂-SO₂-NR₅₂₂-R₆₂₂-alkenyl;
   -R₄₂₂-NR₃₂₂-SO₂-NR₅₂₂-R₆₂₂-aryl;
   -R₄₂₂-NR₃₂₂-SO₂-NR₅₂₂-R₆₂₂-heteroaryl;
   -R₄₂₂-NR₃₂₂-SO₂-NR₅₂₂-R₆₂₂-heterocyclyl; and
   -R₄₂₂-NR₃₂₂-SO₂-N-H₂;
R₂₂₂ is selected from:
   -hydrogen;
   -alkyl;
   -alkenyl;
   -aryl;
   -heteroaryl;
   -heterocyclyl;
   -alkyl-Y-alkyl;
   -alkyl-Y- alkenyl;
   -alkyl-Y-aryl; and
   - alkyl or alkenyl substituted by one or more substituents selected from:
      -OH;
      -halogen;
      -N(R₅₂₂)₂;
      -CO-N(R₅₂₂)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
Y is -O- or -S(O)₀₋₂-;
R₃₂₂ is H, C₁₋₁₀ alkyl, or arylalkyl; each R₄₂₂ is independently alkyl or alkenyl, which may be interrupted by one or more -O- groups; or R₃₂₂ and R₄₂₂ can join together to form a ring;
each R₅₂₂ is independently H, C₁₋₁₀ alkyl, or C₂₋₁₀ alkenyl;
R₆₂₂ is a bond, alkyl, or alkenyl, which may be interrupted by one or more -O- groups;
R₇₂₂ is C₁₋₁₀ alkyl; or R₃₂₂ and R₇₂₂ can join together to form a ring;
v is 0 to 4; and
each R₂₂ present is independently selected from C₁₋₁₀ alkyl, C₁₋₁₀ alkoxy, hydroxy, halogen, and trifluoromethyl;

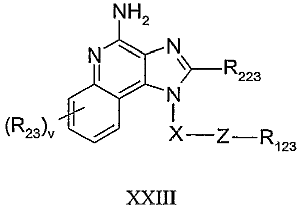

wherein: X is -CHR₃₂₃-, -CHR₃₂₃-alkyl-, or -CHR₃₂₃-alkenyl-;
Z is -S-, -SO-, or -SO₂-;
R₁₂₃ is selected from:
   -alkyl;
   -aryl;
   -heteroaryl;
   -heterocyclyl;
   -alkenyl;
   -R₄₂₃-aryl;
   -R₄₂₃- heteroaryl; and
   -R₄₂₃-heterocyclyl;
R₂₂₃ is selected from:
   -hydrogen;
   -alkyl;
   -alkenyl;
   -aryl;
   -heteroaryl;
   -heterocyclyl;
   -alkyl-Y-alkyl;
   - alkyl-Y- alkenyl;
   -alkyl-Y-aryl; and
   - alkyl or alkenyl substituted by one or more substituents selected from:
      -OH;
      -halogen;
      -N(R₃₂₃)₂;
      -CO-N(R₃₂₃)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
each R₃₂₃ is independently H or C₁₋₁₀ alkyl;
each R₄₂₃ is independently alkyl or alkenyl;
each Y is independently -O- or -S(O)₀₋₂-;
v is 0 to 4; and
each R₂₃ present is independently selected from C₁₋₁₀ alkyl, C₁₋₁₀ alkoxy, hydroxy, halogen, and trifluoromethyl;

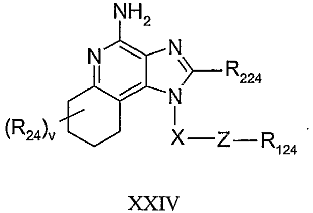

wherein: X is -CHR₃₂₄-, -CHR₃₂₄-alkyl-, or -CHR₃₂₄-alkenyl-;
Z is -S-, -SO-, or -SO₂-;
R₁₂₄ is selected from:
   -alkyl;
   -aryl;
   -heteroaryl;
   -heterocyclyl;
   -alkenyl;
   -R₄₂₄-aryl;
   -R₄₂₄-heteroaryl; and
   -R₄₂₄-heterocyclyl;
R₂₂₄ is selected from:
   -hydrogen;
   -alkyl;
   -alkenyl;
   -aryl;
   -heteroaryl;
   -heterocyclyl;
   -alkyl-Y-alkyl;
   - alkyl-Y-alkenyl;
   -alkyl-Y-aryl; and
   - alkyl or alkenyl substituted by one or more substituents selected from:
      -OH;
      -halogen;
      -N(R₃₂₄)₂;
      -CO-N(R₃₂₄)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
each R₃₂₄ is independently H or C₁₋₁₀ alkyl;
each R₄₂₄ is independently alkyl or alkenyl;
each Y is independently-O- or -S(O)₀₋₂-;
v is 0 to 4; and
each R₂₄ present is independently selected from C₁₋₁₀ alkyl, C₁₋₁₀ alkoxy, hydroxy, halogen, and trifluoromethyl;

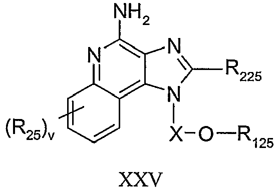

wherein: X is -CHR₅₂₅-, -CHR₅₂₅-alkyl-, or -CHR₅₂₅-alkenyl-;
R₁₂₅ is selected from:
   -R₄₂₅-NR₈₂₅-CR₃₂₅-NR₅₂₅-Z-R₆₂₅-alkyl;
   -R₄₂₅-NR₈₂₅-CR₃₂₅-NR₅₂₅-Z-R₆₂₅-alkenyl;
   -R₄₂₅-NR₈₂₅-CR₃₂₅-NR₅₂₅-Z-R₆₂₅-aryl;
   -R₄₂₅-NR₈₂₅-CR₃₂₅-NR₅₂₅-Z-R₆₂₅-heteroaryl;
   -R₄₂₅-NR₈₂₅-CR₃₂₅-NR₅₂₅-Z-R₆₂₅-heterocyclyl;
   -R₄₂₅-NR₈₂₅-CR₃₂₅-NR₅₂₅R₇₂₅;
   -R₄₂₅-NR₈₂₅-CR₃₂₅-NR₉₂₅-Z-R₆₂₅-alkyl;
   -R₄₂₅-NR₈₂₅-CR₃₂₅-NR₉₂₅-Z-R₆₂₅-alkenyl;
   -R₄₂₅-NR₈₂₅-CR₃₂₅₋NR₉₂₅-Z-R₆₂₅-aryl;
   -R₄₂₅-NR₈₂₅-CR₃₂₅-NR₉₂₅-Z-R₆₂₅-heteroaryl; and
   -R₄₂₅-NR₈₂₅-CR₃₂₅-NR₉₂₅-Z-R₆₂₅-heterocyclyl;
R₂₂₅ is selected from:
   -hydrogen;
   -alkyl;
   -alkenyl;
   -aryl;
   -heteroaryl;
   -heterocyclyl;
   -alkyl-Y-alkyl;
   -alkyl-Y- alkenyl;
   -alkyl-Y-aryl; and
   - alkyl or alkenyl substituted by one or more substituents selected from:
      -OH;
      -halogen;
      -N(R₅₂₅)₂;
      -CO-N(R₅₂₅)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
each R₃₂₅ is =O or =S;
each R₄₂₅ is independently alkyl or alkenyl, which may be interrupted by one or more -O- groups;
each R₅₂₅ is independently H or C₁₋₁₀ alkyl;
R₆₂₅ is a bond, alkyl, or alkenyl, which may be interrupted by one or more -O- groups;
R₇₂₅ is H or C₁₋₁₀ alkyl which may be interrupted by a hetero atom, or R₇₂₅ can join with R₅₂₅ to form a ring;
R₈₂₅ is H, C₁₋₁₀ alkyl, or arylalkyl; or R₄₂₅ and R₈₂₅ can join together to form a ring;
R₉₂₅ is C₁₋₁₀ alkyl which can join together with R₈₂₅ to form a ring;
each Y is independently -O- or -S(O)₀₋₂-;
Z is a bond, -CO-, or -SO₂-;
v is 0 to 4; and
each R₂₅ present is independently selected C₁₋₁₀ alkyl, C₁₋₁₀ alkoxy, hydroxy, halogen, and trifluoromethyl;

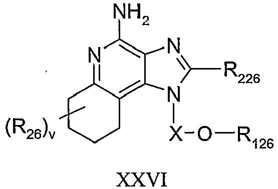

wherein: X is -CHR₅₂₆-, -CHR₅₂₆-alkyl-, or -CHR₅₂₆-alkenyl-;
R₁₂₆ is selected from:
   -R₄₂₆-NR₈₂₆-CR₃₂₆-NR₅₂₆-Z-R₆₂₆-alkyl;
   -R₄₂₆-NR₈₂₆-CR₃₂₆-NR₅₂₆-Z-R₆₂₆-alkenyl;
   -R₄₂₆-NR₈₂₆-CR₃₂₆-NR₅₂₆-Z-R₆₂₆-aryl;
   -R₄₂₆-NR₈₂₆-CR₃₂₆-NR₅₂₆-Z-R₆₂₆-heteroaryl;
   -R₄₂₆-NR₈₂₆-CR₃₂₆-NR₅₂₆-Z-R₆₂₆-heterocyclyl;
   -R₄₂₆-NR₈₂₆-CR₃₂₆-NR₅₂₆R₇₂₆;
   -R₄₂₆-NR₈₂₆-CR₃₂₆-NR₉₂₆-Z-R₆₂₆-alkyl;
   -R₄₂₆-NR₈₂₆-CR₃₂₆-NR₉₂₆-Z-R₆₂₆-alkenyl;
   -R₄₂₆-NR₈₂₆-CR₃₂₆-NR₉₂₆-Z-R₆₂₆-aryl;
   -R₄₂₆-NR₈₂₆-CR₃₂₆-NR₉₂₆-Z-R₆₂₆-heteroaryl; and
   -R₄₂₆-NR₈₂₆-CR₃₂₆-NR₉₂₆-Z-R₆₂₆-heterocyclyl;
R₂₂₆ is selected from:
   -hydrogen;
   -alkyl;
   -alkenyl;
   -aryl;
   -heteroaryl;
   -heterocyclyl;
   -alkyl-Y-alkyl;
   -alkyl-Y-alkenyl;
   -alkyl-Y-aryl; and
   - alkyl or alkenyl substituted by one or more substituents selected from:
      -OH;
      -halogen;
      -N(R₅₂₆)₂;
      -CO-N(R₅₂₆)₂;
      -CO-C₁₋₁₀ alkyl;
      -CO-O-C₁₋₁₀ alkyl;
      -N₃;
      -aryl;
      -heteroaryl;
      -heterocyclyl;
      -CO-aryl; and
      -CO-heteroaryl;
each R₃₂₆ is =O or =S;
each R₄₂₆ is independently alkyl or alkenyl, which may be interrupted by one or more -O- groups;
each R₅₂₆ is independently H or C₁₋₁₀ alkyl;
R₆₂₆ is a bond, alkyl, or alkenyl, which may be interrupted by one or more -O- groups;
R₇₂₆ is H or C₁₋₁₀ alkyl which may be interrupted by a hetero atom, or R₇₂₆ can join with R₅₂₆ to form a ring;
R₈₂₆ is H, C₁₋₁₀ alkyl, or arylalkyl; or R₄₂₆ and R₈₂₆ can join together to form a ring;
R₉₂₆ is C₁₋₁₀ alkyl which can join together with R₈₂₆ to form a ring;
each Y is independently -O- or -S(O)₀₋₂-;
Z is a bond, -CO-, or -SO₂-;
v is 0 to 4; and
each R₂₆ present is independently selected from C₁₋₁₀ alkyl, C₁₋₁₀ alkoxy, hydroxy, halogen, and trifluoromethyl;
and pharmaceutically acceptable salts of any of the foregoing.

Still further disclosed IRM compounds can be chosen from 1*H-*imidazo[4,5-*c*]pyridin-4-amines defined by Formula XXVII below: 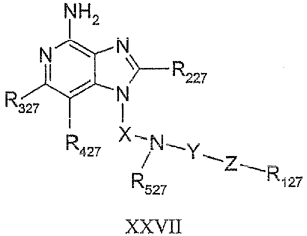
wherein X is alkylene or alkenylene;
Y is -CO- or -CS:
Z is a bond, -O-, or -S-;
R₁₂₇ is aryl, heteroaryl, heterocyclyl, alkyl or alkenyl, each of which may be unsubstituted or substituted by one or more substituents independently selected from:
   -alkyl;
   -alkenyl;
   -aryl;
   -heteroaryl;
   -heterocyclyl;
   -substituted cycloalkyl;
   -substituted aryl;
   -substituted heteroaryl;
   -substituted heterocyclyl;
   -O-alkyl;
   -O-(alkyl)₀₋₁-aryl;
   -O-(alkyl)₀₋₁-(substituted aryl);
   -O-(alkyl)₀₋₁-heteroaryl;
   -O-(alkyl)₀₋₁-(substituted heteroaryl);
   -O-(alkyl)₀₋₁-heterocyclyl;
   -O-(alkyl)₀₋₁-(substituted heterocyclyl);
   -COOH;
   -CO-O-alkyl;
   -CO-alkyl;
   -S(O)₀₋₂-alkyl;
   -S(O)₀₋₂-(alkyl)₀₋₁-aryl;
   -S(O)₀₋₂-(alkyl)₀₋₁-(substituted aryl);
   -S(O)₀₋₂-(alkyl)₀₋₁-heteroaryl;
   -S(O)₀₋₂-(alkyl)₀₋₁-(substituted heteroaryl);
   -S(O)₀₋₂-(alkyl)₀₋₁-heterocyclyl;
   -S(O)₀₋₂-(alkyl)₀₋₁-(substituted heterocyclyl);
   -(alkyl)₀₋₁-N(R₆₂₇)₂;
   -(alkyl)₀₋₁-NR₆₂₇-CO-O-alkyl;
   -(alkyl)₀₋₁-NR₆₂₇-CO-alkyl;
   -(alkyl)₀₋₁-NR₆₂₇-CO-aryl;
   -(alkyl)₀₋₁-NR₆₂₇-CO-(substituted aryl);
   -(alkyl)₀₋₁-NR₆₂₇-CO-heteroaryl;
   -(alkyl)₀₋₁-NR₆₂₇-CO-(substituted heteroaryl);
   -N₃;
   -halogen;
   -haloalkyl;
   -haloalkoxy;
   -CO-haloalkyl;
   -CO-haloalkoxy;
   -NO₂;
   -CN;
   -OH;
   -SH; and in the case of alkyl, alkenyl, and heterocyclyl, oxo;
R₂₂₇ is selected from:
   -hydrogen;
   -alkyl;
   -alkenyl;
   -aryl;
   -substituted aryl;
   -heteroaryl;
   -substituted heteroaryl;
   -alkyl-O-alkyl;
   -alkyl-S-alkyl;
   -alkyl-O-aryl;
   -alkyl-S-aryl:
   -alkyl-O- alkenyl;
   -alkyl-S- alkenyl; and
   -alkyl or alkenyl substituted by one or more substituents selected from:
      -OH;
      -halogen;
      -N(R₆₂₇)₂;
      -CO-N(R₆₂₇)₂;
      -CS-N(R₆₂₇)₂;
      -SO₂-N(R₆₂₇)₂;
      -NR₆₂₇-CO-C₁₋₁₀ alkyl;
      -NR₆₂₇-CS-C₁₋₁₀ alkyl;
      -NR₆₂₇-SO₂-C₁₋₁₀ alkyl;
      -CO-C₁₋₁₀ alkyl;
      -CO-O-C₁₋₁₀ alkyl;
      -N₃;
      -aryl;
      -substituted aryl;
      -heteroaryl;
      -substituted heteroaryl;
      -heterocyclyl;
      -substituted heterocyclyl;
      -CO-aryl;
      -CO-(substituted aryl);
      -CO-heteroaryl; and
      -CO-(substituted heteroaryl);
R₃₂₇ and R₄₂₇ are independently selected from hydrogen, alkyl, alkenyl, halogen, alkoxy, amino, alkylamino, dialkylamino, and alkylthio;
R₅₂₇ is H or C₁₋₁₀ alkyl, or R₅₂₇ can join with X to form a ring that contains one or two heteroatoms; or when R₁₂₇ is alkyl, R₅₂₇ and R₁₂₇ can join to form a ring;
each R₆₂₇ is independently H or C₁₋₁₀alkyl;
and pharmaceutically acceptable salts thereof.

Other disclosed IRM compounds can be chosen from 1*H-*imidazo[4,5-*c*]pyridin-4-amines defined by Formula XXVIII below: 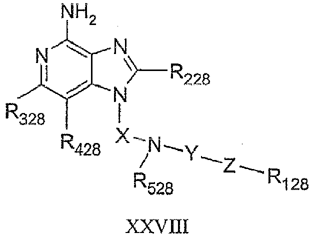
wherein X is alkylene or alkenylene;
Y is -SO₂-;
Z is a bond or -NR₆₂₈-;
R₁₂₈ is aryl, heteroaryl, heterocyclyl alkyl or alkenyl, each of which may be unsubstituted or substituted by one or more substituents independently selected from:
   -alkyl;
   -alkenyl;
   -aryl;
   -heteroaryl;
   -heterocyclyl;
   -substituted cycloalkyl;
   -substituted aryl;
   -substituted heteroaryl;
   -substituted heterocyclyl;
   -O-alkyl;
   -O-(alkyl)₀₋₁-aryl;
   -O-(alkyl)₀₋₁-(substituted aryl);
   -O-(alkyl)₀₋₁-heteroaryl;
   -O-(alkyl)₀₋₁-(substituted heteroaryl);
   -O-(alkyl)₀₋₁-heterocyclyl;
   -O-(alkyl)₀₋₁-(substituted heterocyclyl);
   -COOH;
   -CO-O-alkyl;
   -CO-alkyl;
   -S(O)₀₋₂-alkyl;
   -S(O)₀₋₂-(alkyl)₀₋₁-aryl;
   -S(O)₀₋₂-(alkyl)₀₋₁-(substituted aryl);
   -S(O)₀₋₂-(alkyl)₀₋₁-heteroaryl;
   -S(O)₀₋₂-(alkyl)₀₋₁-(substituted heteroaryl);
   -S(O)₀₋₂-(alkyl)₀₋₁-heterocyclyl;
   -S(O)₀₋₂-(alkyl)₀₋₁-(substituted heterocyclyl);
   -(alkyl)₀₋₁-N(R₆₂₈)₂;
   -(alkyl)₀₋₁-NR₆₂₈-CO-O-alkyl;
   -(alkyl)₀₋₁-NR₆₂₈-CO-alkyl;
   -(alkyl)₀₋₁-NR₆₂₈-CO-aryl;
   -(alkyl)₀₋₁-NR₆₂₈-CO-(substituted aryl);
   -(alkyl)₀₋₁-NR₆₂₈-CO-heteroaryl;
   -(alkyl)₀₋₁-NR₆₂₈-CO-(substituted heteroaryl);
   -N₃;
   -halogen;
   -haloalkyl;
   -haloalkoxy;
   -CO-haloalkyl;
   -CO-haloalkoxy;
   -NO₂;
   -CN;
   -OH;
   -SH; and in the case of alkyl, alkenyl, and heterocyclyl, oxo;
R₂₂₈ is selected from:
   -hydrogen;
   -alkyl;
   -alkenyl;
   -aryl;
   -substituted aryl;
   -heteroaryl;
   -substituted heteroaryl;
   -alkyl-O-alkyl;
   -alkyl-S-alkyl;
   -alkyl-O-aryl;
   -alkyl-S-aryl:
   -alkyl-O- alkenyl;
   -alkyl-S- alkenyl; and
   -alkyl or alkenyl substituted by one or more substituents selected from:
      -OH;
      -halogen;
      -N(R₆₂₈)₂,
      -CO-N(R₆₂₈)₂;
      -CS-N(R₆₂₈)₂;
      -SO₂-N(R₆₂₈)₂;
      -NR₆₂₈-CO-C₁₋₁₀ alkyl;
      -NR₆₂₈-CS-C₁₋₁₀ alkyl;
      -NR₆₂₈-SO₂-C₁₋₁₀ alkyl;
      -CO-C₁₋₁₀ alkyl;
      -CO-O-C₁₋₁₀ alkyl;
      -N₃;
      -aryl;
      -substituted aryl;
      -heteroaryl;
      -substituted heteroaryl;
      -heterocyclyl;
      -substituted heterocyclyl;
      -CO-aryl;
      -CO-(substituted aryl);
      -CO-heteroaryl; and
      -CO-(substituted heteroaryl);
R₃₂₈ and R₄₂₈ are independently selected from hydrogen, alkyl, alkenyl, halogen, alkoxy, amino, alkylamino, dialkylamino,, and alkylthio;
R₅₂₈ is H or C₁₋₁₀ alkyl, or R₅₂₈ can join with X to form a ring; or when R₁₂₈ is alkyl, R₅₂₈ and R₁₂₈ can join to form a ring;
each R₆₂₈ is independently H or C₁₋₁₀alkyl; and pharmaceutically acceptable salts thereof.

Other disclosed IRM compounds can be chosen from 1*H-*imidazo[4,5-*c*]pyridin-4-amines defined by Formula XXIX below: 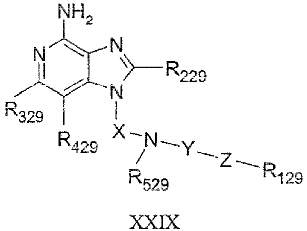
wherein X is alkylene or alkenylene;
Y is -CO- or -CS;
Z is -NR₆₂₉-, -NR₆₂₉-CO-, -NR₆₂₉-SO₂-, or -NR₇₂₉-;
R₁₂₉ is aryl, heteroaryl, heterocyclyl, alkyl or alkenyl, each of which may be unsubstituted or substituted by one or more substituents independently selected from:
   -alkyl;
   -alkenyl;
   -aryl;
   -heteroaryl;
   -heterocyclyl;
   -substituted cycloalkyl;
   -substituted aryl;
   -substituted heteroaryl;
   -substituted heterocyclyl;
   -O-alkyl;
   -O-(alkyl)₀₋₁-aryl;
   -O-(alkyl)₀₋₁-(substituted aryl);
   -O-(alkyl)₀₋₁-heteroaryl;
   -O-(alkyl)₀₋₁-(substituted heteroaryl);
   -O-(alkyl)₀₋₁-heterocyclyl;
   -O-(alkyl)₀₋₁-(substituted heterocyclyl);
   -COOH;
   -CO-O-alkyl;
   -CO-alkyl;
   -S(O)₀₋₂ -alkyl;
   -S(O)₀₋₂-(alkyl)₀₋₁-aryl;
   -S(O)₀₋₂-(alkyl)₀₋₁-(substituted aryl);
   -S(O)₀₋₂-(alkyl)₀₋₁-heteroaryl;
   -S(O)₀₋₂-(alkyl)₀₋₁-(substituted heteroaryl);
   -S(O)₀₋₂-(alkyl)₀₋₁-heterocyclyl;
   -S(O)₀₋₂-(alkyl)₀₋₁-(substituted heterocyclyl);
   -(alkyl)₀₋₁-N(R₆₂₉)₂;
   -(alkyl)₀₋₁-NR₆₂₉-CO-O-alkyl;
   -(alkyl)₀₋₁-NR₆₂₉-CO-alkyl;
   -(alkyl)₀₋₁-NR₆₂₉-CO-aryl;
   -(alkyl)₀₋₁-NR₆₂₉-CO-(substituted aryl);
   -(alkyl)₀₋₁-NR₆₂₉-CO-heteroaryl;
   -(alkyl)₀₋₁-NR₆₂₉-CO-(substituted heteroaryl);
   -P(O)(O-alkyl)₂;
   -N₃;
   -halogen;
   -haloalkyl;
   -haloalkoxy;
   -CO-haloalkyl;
   -CO-haloalkoxy;
   -NO₂;
   -CN;
   -OH;
   -SH; and in the case of alkyl, alkenyl, and heterocyclyl, oxo;
R₂₂₉ is selected from:
   -hydrogen;
   -alkyl;
   -alkenyl;
   -aryl;
   -substituted aryl;
   -heteroaryl;
   -substituted heteroaryl;
   -alkyl-O-alkyl;
   -alkyl-S-alkyl;
   -alkyl-O-aryl;
   -alkyl-S-aryl:
   -alkyl-O- alkenyl;
   -alkyl-S- alkenyl; and
   -alkyl or alkenyl substituted by one or more substituents selected from:
      -OH;
      -halogen;
      -N(R₆₂₉)₂;
      -CO-N(R₆₂₉)₂;
      -CS-N(R₆₂₉)₂;
      -SO₂-N(R₆₂₉)₂;
      -NR₆₂₉-CO-C₁₋₁₀ alkyl;
      -NR₆₂₉-CS-C₁₋₁₀ alkyl;
      -NR₆₂₉-SO₂-C₁₋₁₀ alkyl;
      -CO-C₁₋₁₀ alkyl;
      -CO-O-C₁₋₁₀ alkyl;
      -N₃;
      -aryl;
      -substituted aryl;
      -heteroaryl;
      -substituted heteroaryl;
      -heterocyclyl;
      -substituted heterocyclyl;
      -CO-aryl;
      -CO-(substituted aryl);
      -CO-heteroaryl; and
      -CO-(substituted heteroaryl);
R₃₂₉ and R₄₂₉ are independently selected from hydrogen, alkyl, alkenyl, halogen, alkoxy, amino, alkylamino, dialkylamino, and alkylthio;
R₅₂₉ is H or C₁₋₁₀ alkyl, or R₅₂₉ can join with X to form a ring that contains one or two heteroatoms;
each R₆₂₉ is independently H or C₁₋₁₀alkyl;
R₇₂₉ is H or C₁₋₁₀ alkyl which may be interrupted by a heteroatom; or when R₁₂₉ is alkyl, R₇₂₉ and R₁₂₉ can join to form a ring;
and pharmaceutically acceptable salts thereof.

Other disclosed IRM compounds can be chosen from 1-position ether or thioether substituted 1*H*-imidazo[4,5-*c*]pyridin-4-amines defined by Formula XXX below: 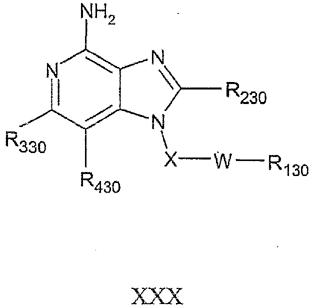 wherein:
X is -CH(R₅₃₀)-, -CH(R₅₃₀)-alkylene-, -CH(R₅₃₀)-alkenylene-, or CH(R₅₃₀)-alkylene-Y-alkylene-;
Y is -O-., or -S(O)₀₋₂-;
   -W-R₁₃₀ is selected from -O-R₁₃₀₋₁₋₅ and -S(O)₀₋₂-R₁₃₀₋₆;
R₁₃₀₋₁₋₅ is selected from
   -R₆₃₀-C(R₇₃₀)-Z-R₈₃₀-alkyl;
   -R₆₃₀-C(R₇₃₀)-Z-R₈₃₀-alkenyl;
   -R₆₃₀-C(R₇₃₀)-Z-R₈₃₀-aryl;
   -R₆₃₀-C(R₇₃₀)-Z-R₈₃₀-heteroaryl;
   -R₆₃₀-C(R₇₃₀)-Z-R₈₃₀-heterocyclyl;
   -R₆₃₀-C(R₇₃₀)-Z-H;
   -R₆₃₀-N(R₉₃₀)-C(R₇₃₀)-R₈₃₀-alkyl;
   -R₆₃₀-N(R₉₃₀)-C(R₇₃₀)-R₈₃₀-alkenyl;
   -R₆₃₀-N(R₉₃₀)-C(R₇₃₀)-R₈₃₀-aryl;
   -R₆₃₀-N(R₉₃₀)-C(R₇₃₀)-R₈₃₀-heteroaryl;
   -R₆₃₀-N(R₉₃₀)-C(R₇₃₀)-R₈₃₀-heterocyclyl;
   -R₆₃₀-N(R₉₃₀)-C(R₇₃₀)-R₁₀₃₀;
   -R₆₃₀-N(R₉₃₀)-SO₂-R₈₃₀-alkyl;
   -R₆₃₀-N(R₉₃₀)-SO₂-R₈₃₀-alkenyl;
   -R₆₃₀-N(R₉₃₀)-SO₂-R₈₃₀-aryl;
   -R₆₃₀-N(R₉₃₀)-SO₂-R₈₃₀-heteroaryl;
   -R₆₃₀-N(R₉₃₀)-SO₂-R₈₃₀-heterocyclyl;
   -R₆₃₀-N(R₉₃₀)-SO₂-R₁₀₃₀;
   -R₆₃₀-N(R₉₃₀)-SO₂-N(R₅₃₀)-R₈₃₀-alkyl;
   -R₆₃₀-N(R₉₃₀)-SO₂-N(R₅₃₀)-R₈₃₀-alkenyl;
   -R₆₃₀-N(R₉₃₀)-SO₂-N(R₅₃₀)-R₈₃₀-aryl;
   -R₆₃₀-N(R₉₃₀)-SO₂-N(R₅₃₀)-R₈₃₀-heteroaryl;
   -R₆₃₀-N(R₉₃₀)-SO₂-N(R₅₃₀)-R₈₃₀-heterocyclyl;
   -R₆₃₀-N(R₉₃₀)-SO₂-NH₂;
   -R₆₃₀-N(R₉₃₀)-C(R₇₃₀)-N(R₅₃₀)-Q-R₈₃₀-alkyl;
   -R₆₃₀-N(R₉₃₀)-C(R₇₃₀)-N(R₅₃₀)-Q-R₈₃₀-alkenyl;
   -R₆₃₀-N(R₉₃₀)-C(R₇₃₀)-N(R₅₃₀)-Q-R₈₃₀-aryl;
   -R₆₃₀-N(R₉₃₀)-C(R₇₃₀)-N(R₅₃₀)-Q-R₈₃₀-heteroaryl;
   -R₆₃₀-N(R₉₃₀)-C(R₇₃₀)-N(R₅₃₀)-Q-R₈₃₀-heterocyclyl;
   -R₆₃₀-N(R₉₃₀)-C(R₇₃₀)-N(R₅₃₀)₂; 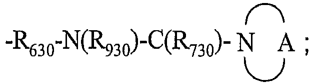
   -R₆₃₀-N(R₉₃₀)-C(R₇₃₀)-N(R₁₁₃₀)-Q-R₈₃₀-alkyl;
   -R₆₃₀-N(R₉₃₀)-C(R₇₃₀)-N(R₁₁₃₀)-Q-R₈₃₀-alkenyl;
   -R₆₃₀-N(R₉₃₀)-C(R₇₃₀)-N(R₁₁₃₀)-Q-R₈₃₀-aryl;
   -R₆₃₀-N(R₉₃₀)-C(R₇₃₀)-N(R₁₁₃₀)-Q-R₈₃₀-heteroaryl;
   -R₆₃₀-N(R₉₃₀)-C(R₇₃₀)-N(R₁₁₃₀)-Q-R₈₃₀-heterocyclyl;
   -R₆₃₀-N(R₉₃₀)-C(R₇₃₀)-N(R₁₁₃₀)H;
   -alkenyl;
   -aryl;
   -R₆₃₀-aryl;
   -heteroaryl;
   -heterocyclyl;
   -R₆₃₀- heteroaryl; and
   -R₆₃₀-heterocyclyl;
Z is-N(R₅₃₀)-, -O-, or -S-;
Q is a bond, -CO-, or -SO₂-;
A represents the atoms necessary to provide a 5- or 6-membered heterocyclic or heteroaromatic ring that contains up to three heteroatoms;
R₁₃₀₋₆ is selected from:
   -alkyl;
   -aryl;
   -heteroaryl;
   -heterocyclyl;
   -alkenyl;
   -R₆₃₀-aryl;
   -R₆₃₀- heteroaryl; and
   -R₆₃₀-heterocyclyl;
each R₅₃₀ is independently hydrogen, C₁₋₁₀ alkyl, or C₂₋₁₀ alkenyl;
R₆₃₀ is alkylene, alkenylene, or alkynylene, which may be interrupted by one or more -O- groups;
R₇₃₀ is =O or =S;
R₈₃₀ is a bond, alkylene, alkenylene, or alkynylene, which may be interrupted by one or more -O- groups;
R₉₃₀ is hydrogen, C₁₋₁₀ alkyl, or arylalkyl; or R₉₃₀ can join together with any carbon atom of R₆₃₀ to form a ring of the formula 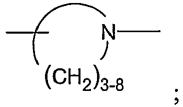
R₁₀₃₀ is hydrogen or C₁₋₁₀ alkyl; or R₉₃₀ and R₁₀₃₀ can join together to form a ring selected from 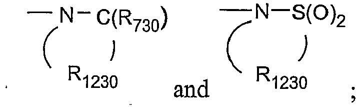
R₁₁₃₀ is C₁₋₁₀ alkyl; or R₉₃₀ and R₁₁₃₀ can join together to form a ring having the structure 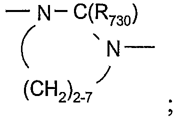
R₁₂₃₀ is C₂₋₇ alkylene which is straight chain or branched, wherein the branching does not prevent formation of the ring; and
R₂₃₀, R₃₃₀ and R₄₃₀ are independently selected from hydrogen and non-interfering substitutents;
and pharmaceutically acceptable salts thereof.

Illustrative non-interfering R₂₃₀ substituents include:
-alkyl;
-alkenyl;
-aryl;
-heteroaryl;
-heterocyclyl;
-alkylene-Y-alkyl;
-alkylene-Y-alkenyl;
-alkylene-Y-aryl; and
- alkyl or alkenyl substituted by one or more substituents selected from the group consisting of:
   -OH;
   -halogen;
   -N(R₅₃₀)₂;
   -C(O)-C₁₋₁₀ alkyl;
   -C(O)-O-C₁₋₁₀ alkyl;
   -N₃;
   -aryl;
   -heteroaryl;
   -heterocyclyl;
   -C(O)-aryl; and
   -C(O)-heteroaryl.

Illustrative non-interfering R₃₃₀ and R₄₃₀ substitutents include:
C₁₋₁₀ alkyl, C₂₋₁₀ alkenyl, C₂₋₁₀ alkynyl, C₁₋₁₀ alkoxy, C₁₋₁₀ alkylthio, amino, alkylamino, dialkylamino, halogen, and nitro.

In another embodiment, the IRM compound can be chosen from 1*H*-imidazo dimers of the formula (XXXI): 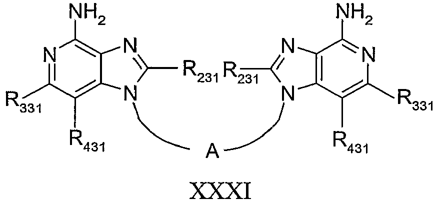 wherein:
A is a divalent linking group selected from the group consisting of:
   straight or branched chain C₄₋₂₀ alkylene;
   straight or branched chain C₄₋₂₀ alkenylene;
   straight or branched chain C₄₋₂₀ alkynylene; and
   -Z-Y-W-Y-Z-;
each Z is independently selected from the group consisting of:
   straight or branched chain C₂₋₂₀ alkylene;
   straight or branched chain C₄₋₂₀ alkenylene; and
   straight or branched chain C₄₋₂₀ alkynylene;
   any of which may be optionally interrupted by -O-, -N(R₅₃₁)-, or -S(O)₂-;
each Y is independently selected from the group consisting of:
   a bond;
   -N(R₅₃₁)C(O)-;
   -C(O)N(R₅₃₁)-;
   -N(R₅₃₁)C(O)N(R₅₃₁)-;
   -N(R₅₃₁)S(O)₂-;
   -S(O)₂N(R₅₃₁)-;
   -OC(O)O-;
   -OC(O)-;
   -C(O)O-;
   -N(R₅₃₁)C(O)O-; and
   -OC(O)N(R₅₃₁)-;
W is selected from the group consisting of:
   straight or branched chain C₂₋₂₀ alkylene;
   straight or branched chain C₂₋₂₀ alkenylene;
   straight or branched chain C₄₋₂₀ alkynylene;
   straight or branched chain perfluoro C₂₋₂₀ alkylene;
   C₁₋₄ alkylene-O-C₁₋₄ alkylene;
   -C(O)-;
   -S(O)₂-;
   -OC(O)O-;
   -N(R₅₃₁)C(O)N(R₅₃₁)-; 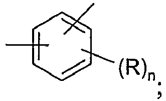 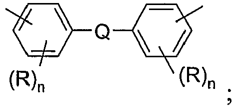 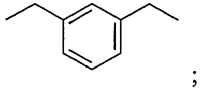
   1,5-naphthylene;
   2,6-pyridinylene;
   1,2-cyclohexylene;
   1,3-cyclohexylene;
   1,4-cyclohexylene;
   trans-1,4-cyclohexylene; 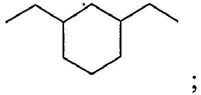 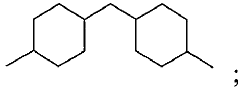 and
   trans-5-norbornen-2,3-diyl;
wherein n is 0 - 4; each R is independently selected from the group consisting of C₁₋₄ alkyl, C₁₋₄ alkoxy, and halogen; and Q is selected from the group consisting of a bond, -CH₂-, and -O-; R₂₃₁ is selected from the group consisting of:
   -hydrogen;
   -alkyl;
   -alkenyl;
   -aryl;
   -substituted aryl;
   -heteroaryl;
   -substituted heteroaryl;
   -alkyl-X-alkyl;
   -alkyl-X-aryl;
   -alkyl-X- alkenyl; and
   -alkyl or alkenyl substituted by one or more substituents selected from the group consisting of:
      -OH;
      -halogen;
      -N(R₆₃₁)₂;
      -C(O)-N(R₆₃₁)₂;
      -C(S)-N(R₆₃₁)₂;
      -S(O)₂-N(R₆₃₁)₂;
      -N(R₆₃₁)-C(O)-C₁₋₁₀ alkyl;
      -N(R₆₃₁)-C(S)-C₁₋₁₀ alkyl;
      -N(R₆₃₁)-S(O)₂-C₁₋₁₀ alkyl;
      -C(O)-C₁₋₁₀ alkyl;
      -C(O)-O-C₁₋₁₀ alkyl;
      -N₃;
      -aryl;
      -substituted aryl;
      -heteroaryl;
      -substituted heteroaryl;
      -heterocyclyl;
      -substituted heterocyclyl;
      -C(O)-aryl;
      -C(O)-(substituted aryl);
      -C(O)-heteroaryl; and
      -C(O)-(substituted heteroaryl);
R₃₃₁ and R₄₃₁ are each independently selected from the group consisting of:
   -hydrogen;
   -halogen;
   -alkyl;
   -alkenyl;
   -X-alkyl; and
   -N(R₆₃₁)₂;
   or when taken together, R₃₃₁ and R₄₃₁ form a fused aryl or heteroaryl ring that is unsubstituted or substituted by one or more substituents selected from the group consisting of:
   -halogen;
   -alkyl;
   -alkenyl;
   -X-alkyl; and
   -N(R₆₃₁)₂;
   or when taken together, R₃₃₁ and R₄₃₁ form a fused 5 to 7 membered saturated ring, containing 0 to 2 heteroatoms and unsubstituted or substituted by one or more substituents selected from the group consisting of:
   -halogen;
   -alkyl;
   -alkenyl;
   -X-alkyl; and
   -N(R₆₃₁)₂;
each R₅₃₁ is independently selected from the group consisting of:
   hydrogen;
   C₁₋₆ alkyl;
   C₃₋₇ cycloalkyl; and
   benzyl; or
when Y is N(R₅₃₁)C(O)-, -C(O)N(R₅₃₁)-, -N(R₅₃₁)C(O)N(R₅₃₁)-, -N(R₅₃₁)S(O)₂, -S(O₂)N(R₅₃₁)-, -N(R₅₃₁)C(O)O-, or -OC(O)N(R₅₃₁)- and the nitrogen of the N(R₅₃₁) group is bonded to Z, then R₅₃₁ can join with Z to form a ring having the structure 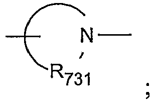
   each R₆₃₁ is independently hydrogen or C₁₋₁₀ alkyl;
   R₇₃₁ is C₃₋₈ alkylene; and
   X is -O- or -S-;
   with the proviso that if W is -C(O)-, -S(O)₂-, -OC(O)O-, or -N(R₅₃₁)C(O)N(R₅₃₁)- then each Y is a bond;
   and pharmaceutically acceptable salts thereof.

Disclosed IRM compounds can be chosen from 6-, 7-, 8-, or 9-position aryl or heteroaryl substituted 1*H*-imidazo[4,5-*c*]quinolin-4-amines of the following Formula (XXXII): 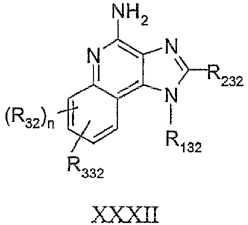 wherein:
R₃₂ is selected from the group consisting of alkyl, alkoxy, hydroxy, and trifluoromethyl;
n is 0 or 1;
R₁₃₂ and R₂₃₂ are independently selected from the group consisting of hydrogen and non-interfering substituents;
R₃₃₂ is selected from the group consisting of:
   -Z-Ar,
   -Z-Ar'-Y-R₄₃₂,
   -Z-Ar'-X-Y-R₄₃₂,
   -Z-Ar'-R₅₃₂, and
   -Z-Ar'-X-R₅₃₂;
Ar is selected from the group consisting of aryl and heteroaryl both of which can be unsubstituted or can be substituted by one or more substituents independently selected from the group consisting of alkyl, alkenyl, alkoxy, methylenedioxy, haloalkyl, haloalkoxy, halogen, nitro, hydroxy, hydroxyalkyl, mercapto, cyano, carboxy, formyl, aryl, aryloxy, arylalkoxy, heteroaryl, heteroaryloxy, heteroarylalkoxy, heterocyclyl, heterocyclylalkyl, amino, alkylamino, and dialkylamino;
Ar' is selected from the group consisting of arylene and heteroarylene both of which can be unsubstituted or can be substituted by one or more substituents independently selected from the group consisting of alkyl, alkenyl, alkoxy, haloalkyl, haloalkoxy, halogen, nitro, hydroxy, hydroxyalkyl, mercapto, cyano, carboxy, formyl, aryl, aryloxy, arylalkoxy, heteroaryl, heteroaryloxy, heteroarylalkoxy, heterocyclyl, heterocyclylalkyl, amino, alkylamino, and dialkylamino;
X is selected from the group consisting of alkylene, alkenylene, alkynylene, arylene, heteroarylene, and heterocyclylene wherein the alkylene, alkenylene, and alkynylene groups can be optionally interrupted or terminated with arylene, heteroarylene, or heterocyclylene, and optionally interrupted by one or more -O-groups;
Y is selected from the group consisting of:
   -S(O)₀₋₂-,
   -S(O)₂-N(R₈₃₂)-,
   -C(R₆₃₂)-,
   -C(R₆₃₂)-O-,
   -O-C(R₆₃₂)-,
   -O-C(O)-O-,
   -N(R₈₃₂)-Q-,
   -C(R₆₃₂)-N(R₈₃₂)-,
   -O-C(R₆₃₂)-N(R₈₃₂)-,
   -C(R₆₃₂)-N(OR₉₃₂)-, 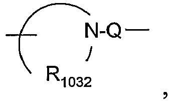 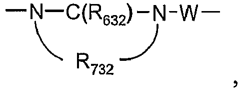 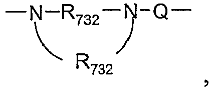 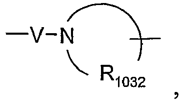 and 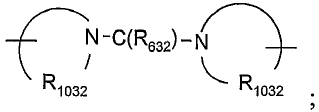
Z is selected from the group consisting of a bond, alkylene, alkenylene, and alkynylene;
R₄₃₂ is selected from the group consisting of hydrogen, alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl wherein the alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl groups can be unsubstituted or substituted by one or more substituents independently selected from the group consisting of alkyl, alkoxy, hydroxyalkyl, haloalkyl, haloalkoxy, halogen, nitro, hydroxy, mercapto, cyano, aryl, aryloxy, arylalkyleneoxy, heteroaryl, heteroaryloxy, heteroarylalkyleneoxy, heterocyclyl, amino, alkylamino, dialkylamino, (dialkylamino)alkyleneoxy, and in the case of alkyl, alkenyl, alkynyl, and heterocyclyl, oxo;
R₅₃₂ is selected from the group consisting of: 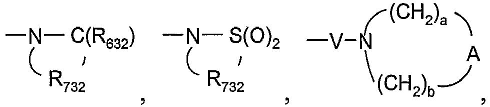 and 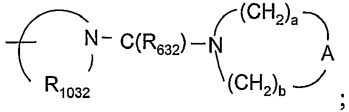
each R₆₃₂ is independently selected from the group consisting of =O and =S;
each R₇₃₂ is independently C₂₋₇ alkylene;
each R₈₃₂ is independently selected from the group consisting of hydrogen, alkyl, alkoxyalkylenyl, and arylalkylenyl;
R₉₃₂ is selected from the group consisting of hydrogen and alkyl; each R₁₀₃₂ is independently C₃₋₈ alkylene;
A is selected from the group consisting of -O-, -C(O)-, -S(O)₀₋₂-, -CH₂-, and - N(R₄₃₂)-,
Q is selected from the group consisting of a bond, -C(R₆₃₂)-, -C(R₆₃₂)-C(R₆₃₂), -S(O)₂-, -C(R₆₃₂)-N(R₈₃₂)-W-, -S(O)₂-N(R₈₃₂)-, -C(R₆₃₂)-O-, and - C(R₆₃₂)-N(OR₉₃₂)-;
V is selected from the group consisting of -C(R₆₃₂)-, -O-C(R₆₃₂)-, -N(R₈₃₂)-C(R₆₃₂)-, and -S(O)₂-;
W is selected from the group consisting of a bond, -C(O)-, and -S(O)₂-; and
a and b are independently integers from 1 to 6 with the proviso that a + b is ≤ 7; and pharmaceutically acceptable salts thereof.

Illustrative non-interfering R₁₃₂ substituents include:
-R₄₃₂,
-X-R₄₃₂,
-X-Y-R₄₃₂,
-X-Y-X-Y-R₄₃₂, and
-X-R₅₃₂;
wherein:
each X is independently selected from the group consisting of alkylene, alkenylene, alkynylene, arylene, heteroarylene, and heterocyclylene wherein the alkylene, alkenylene, and alkynylene groups can be optionally interrupted or terminated with arylene, heteroarylene, or heterocyclylene, and optionally interrupted by one or more -O- groups;
each Y is independently selected from the group consisting of:
   -S(O)₀₋₂-,
   -S(O)₂-N(R₈₃₂)-,
   -C(R₆₃₂)-,
   -C(R₆₃₂)-O-,
   -O-C(R₆₃₂)-,
   -O-C(O)-O-,
   -N(R₈₃₂)-Q-,
   -C(R₆₃₂)-N(R₈₃₂)-,
   -O-C(R₆₃₂)-N(R₈₃₂)-,
   -C(R₆₃₂)-N(OR₉₃₂)-, 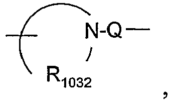 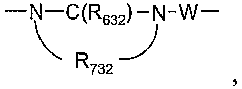 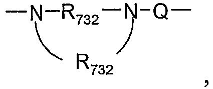 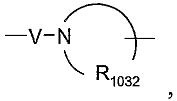 and 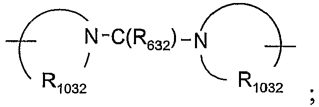
R₄₃₂ is selected from the group consisting of hydrogen, alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl wherein the alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl groups can be unsubstituted or substituted by one or more substituents independently selected from the group consisting of alkyl, alkoxy, hydroxyalkyl, haloalkyl, haloalkoxy, halogen, nitro, hydroxy, mercapto, cyano, aryl, aryloxy, arylalkyleneoxy, heteroaryl, heteroaryloxy, heteroarylalkyleneoxy, heterocyclyl, amino, alkylamino, dialkylamino, (dialkylamino)alkyleneoxy, and in the case of alkyl, alkenyl, alkynyl, and heterocyclyl, oxo;
R₅₃₂ is selected from the group consisting of: 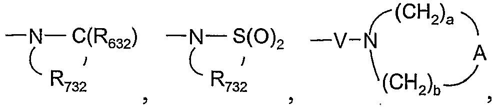 and 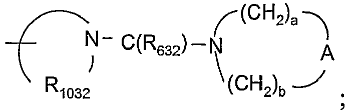
each R₆₃₂ is independently selected from the group consisting of =O and =S;
each R₇₃₂ is independently C₂₋₇ alkylene;
each R₈₃₂ is independently selected from the group consisting of hydrogen, alkyl, alkoxyalkylenyl, and arylalkylenyl;
each R₉₃₂ is independently selected from the group consisting of hydrogen and alkyl;
each R₁₀₃₂ is independently C₃₋₈ alkylene;
A is selected from the group consisting of -O-, -C(O)-, -S(O)₀₋₂-, -CH₂-, and - N(R₄₃₂)-;
each Q is independently selected from the group consisting of a bond, -C(R₆₃₂)-, -C(R₆₃₂)-C(R₆₃₂)-, -S(O)₂-, -C(R₆₃₂)-N(R₈₃₂)-W-, -S(O)₂-N(R₈₃₂)-, -C(R₆₃₂)-O-, and -C(R₆₃₂)-N(OR₉₃₂)-;
each V is independently selected from the group consisting of -C(R₆₃₂)-, -O-C(R₆₃₂)-, -N(R₈₃₂)-C(R₆₃₂)-, and -S(O)₂-;
each W is independently selected from the group consisting of a bond, -C(O)-, and -S(O)₂-; and
a and b are independently integers from 1 to 6 with the proviso that a + b is ≤ 7;

Illustrative non-interfering R₂₃₂ substitutents include:
-R₄₃₂,
-X-R₄₃₂,
-X-Y-R₄₃₂, and
-X-R₅₃₂;
wherein:
X is selected from the group consisting of alkylene, alkenylene, alkynylene, arylene, heteroarylene, and heterocyclylene wherein the alkylene, alkenylene, and alkynylene groups can be optionally interrupted or terminated with arylene, heteroarylene, or heterocyclylene, and optionally interrupted by one or more -O-groups;
Y is selected from the group consisting of:
   -S(O)₀₋₂-,
   -S(O)₂-N(R₈₃₂)-,
   -C(R₆₃₂)-,
   -C(R₆₃₂)-O-,
   -O-C(R₆₃₂)-,
   -O-C(O)-O-,
   -N(R₈₃₂)-Q-,
   -C(R₆₃₂)-N(R₈₃₂)-,
   -O-C(R₆₃₂)-N(R₈₃₂)-,
   -C(R₆₃₂)-N(OR₉₃₂)-, 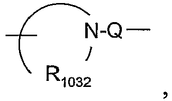 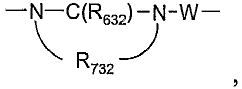 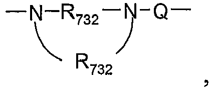 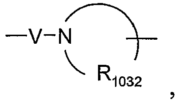 and 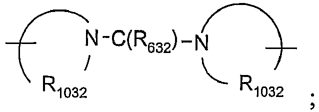
R₄₃₂ is selected from the group consisting of hydrogen, alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl wherein the alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl groups can be unsubstituted or substituted by one or more substituents independently selected from the group consisting of alkyl, alkoxy, hydroxyalkyl, haloalkyl, haloalkoxy, halogen, nitro, hydroxy, mercapto, cyano, aryl, aryloxy, arylalkyleneoxy, heteroaryl, heteroaryloxy, heteroarylalkyleneoxy, heterocyclyl, amino, alkylamino, dialkylamino, (dialkylamino)alkyleneoxy, and in the case of alkyl, alkenyl, alkynyl, and heterocyclyl, oxo;
R₅₃₂ is selected from the group consisting of: 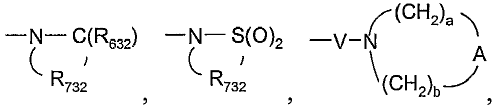 and 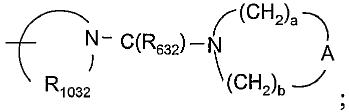
each R₆₃₂ is independently selected from the group consisting of =O and =S;
each R₇₃₂ is independently C₂₋₇ alkylene;
each R₈₃₂ is independently selected from the group consisting of hydrogen, alkyl, alkoxyalkylenyl, and arylalkylenyl;
R₉₃₂ is selected from the group consisting of hydrogen and alkyl;
each R₁₀₃₂ is independently C₃₋₈ alkylene;
A is selected from the group consisting of -O-, -C(O)-, -S(O)₀₋₂-, -CH₂-, and - N(R₄₃₂)-;
Q is selected from the group consisting of a bond, -C(R₆₃₂)-, -C(R₆₃₂)-C(R₆₃₂)-, -S(O)₂-, -C(R₆₃₂)-N(R₈₃₂)-W-, -S(O)₂-N(R₈₃₂)-, -C(R₆₃₂)-O-, and-C(R₆₃₂)-N(OR₉₃₂)-;
V is selected from the group consisting of -C(R₆₃₂)-, -O-C(R₆₃₂)-, -N(R₈₃₂)-C(R₆₃₂)-, and -S(O)₂-;
W is selected from the group consisting of a bond, -C(O)-, and -S(O)₂-; and
a and b are independently integers from 1 to 6 with the proviso that a + b is ≤ 7;

Herein, "non-interfering" means that the ability of the compound or salt to modulate (e.g., induce or inhibit) the biosynthesis of one or more cytokines is not destroyed by the non-interfering substituent.

As used herein, the terms "alkyl", "alkenyl", "alkynyl" and the prefix "alk-" are inclusive of both straight chain and branched chain groups and of cyclic groups, i.e. cycloalkyl and cycloalkenyl. Unless otherwise specified, these groups contain from 1 to 20 carbon atoms, with alkenyl and alkynyl groups containing from 2 to 20 carbon atoms. In some embodiments, these groups have a total of up to 10 carbon atoms, up to 8 carbon atoms, up to 6 carbon atoms, or up to 4 carbon atoms. Cyclic groups can be monocyclic or polycyclic and preferably have from 3 to 10 ring carbon atoms. Exemplary cyclic groups include cyclopropyl, cyclopropylmethyl, cyclopentyl, cyclohexyl, adamantyl, and substituted and unsubstituted bornyl, norbornyl, and norbornenyl.

Unless otherwise specified, "alkylene", "alkenylene", and "alkynylene" are the divalent forms of the "alkyl", "alkenyl", and "alkynyl" groups defined above. For example, an arylalkenyl group comprises an alkylene moiety to which an aryl group is attached.

The term "haloalkyl" is inclusive of groups that are substituted by one or more halogen atoms, including perfluorinated groups. This is also true of other groups that include the prefix "halo-". Examples of suitable haloalkyl groups are chloromethyl, trifluoromethyl, and the like.

The term "aryl" as used herein includes carbocyclic aromatic rings or ring systems. Examples of aryl groups include phenyl, naphthyl, biphenyl, fluorenyl, and indenyl.

The term "hetero atom" refers to the atoms O, S, or N.

The term "heteroaryl" includes aromatic rings or ring systems that contain at least one ring hetero atom. Suitable heteroaryl groups include furyl, thienyl, pyridyl, quinolinyl, isoquinolinyl, indolyl, isoindolyl, triazolyl, pyrrolyl, tetrazolyl, imidazolyl, pyrazolyl, oxazolyl, thiazolyl, benzofuranyl, benzothiophenyl, carbazolyl, benzoxazolyl, pyrimidinyl, benzimidazolyl, quinoxalinyl, benzothiazolyl, naphthyridinyl, isoxazolyl, isothiazolyl, purinyl, quinazolinyl, pyrazinyl, 1-oxidopyridyl, pyridazinyl, triazinyl, tetrazinyl, oxadiazolyl, thiadiazolyl, and so on.

The term "heterocyclyl" includes non-aromatic rings or ring systems that contain at least one ring hetero atom and includes all of the fully saturated and partially unsaturated derivatives of the above mentioned heteroaryl groups. Exemplary heterocyclic groups include pyrrolidinyl, tetrahydrofuranyl, morpholinyl, thiomorpholinyl, piperidinyl, piperazinyl, thiazolidinyl, imidazolidinyl, isothiazolidinyl, tetrahydropyranyl, quinuclidinyl, homopiperidinyl, homopiperazinyl, and the like.

The terms "arylene," "heteroarylene," and "heterocyclylene" are the divalent forms of the "aryl," "heteroaryl," and "heterocyclyl" groups defined above. Likewise, "arylenyl," "heteroarylenyl," and "heterocyclylenyl" are the divalent forms of the "aryl," "heteroaryl," and "heterocyclyl" groups defined above. For example, an alkylarylenyl group comprises an arylene moiety to which an alkyl group is attached.

Unless otherwise specified, the aryl, heteroaryl, and heterocyclyl groups of Formulas IX - XXX can be unsubstituted or substituted by one or more substituents independently selected from the group consisting of alkyl, alkoxy, methylenedioxy, ethylenedioxy, alkylthio, haloalkyl, haloalkoxy, haloalkylthio, halogen, nitro, hydroxy, mercapto, cyano, carboxy, formyl, aryl, aryloxy, arylthio, arylalkoxy, arylalkylthio, heteroaryl, heteroaryloxy, heteroarylthio, heteroarylalkoxy, heteroarylalkylthio, amino, alkylamino, dialkylamino, heterocyclyl, heterocycloalkyl, alkylcarbonyl, alkenylcarbonyl, alkoxycarbonyl, haloalkylcarbonyl, haloalkoxycarbonyl, alkylthiocarbonyl, arylcarbonyl, heteroarylcarbonyl, heterocyclylcarbonyl, aryloxycarbonyl, heteroaryloxycarbonyl, arylthiocarbonyl, heteroarylthiocarbonyl, alkanoyloxy, alkanoylthio, alkanoylamino, aroyloxy, aroylthio, aroylamino, alkylaminosulfonyl, alkylsulfonyl, arylsulfonyl, heteroarylsulfonyl, aryldiazinyl, alkylsulfonylamino, arylsulfonylamino, arylalkylsulfonylamino, alkylcarbonylamino, alkenylcarbonylamino, arylcarbonylamino, arylalkylcarbonylamino, heteroarylcarbonylamino, heteroarylalkycarbonylamino, alkylsulfonylamino, alkenylsulfonylamino, arylsulfonylamino, arylalkylsulfonylamino, heteroarylsulfonylamino, heteroarylalkylsulfonylamino, alkylaminocarbonyl, dialkylaminocarbonyl, arylaminocarbonyl, arylalkylaminocarbonyl, alkenylaminocarbonyl, heteroarylaminocarbonyl, heteroarylalkylaminocarbonyl, alkylaminocarbonylamino, alkenylaminocarbonylamino, arylaminocarbonylamino, arylalkylaminocarbonylamino, heteroarylaminocarbonylamino, heteroarylalkylaminocarbonylamino and, in the case of heterocyclyl, oxo. If any other groups are identified as being "substituted" or "optionally substituted", then those groups can also be substituted by one or more of the above enumerated substituents.

The IRM compounds and salts thereof described herein include any of their pharmaceutically acceptable forms, such as isomers (e.g., diastereomers and enantiomers), solvates, polymorphs, and the like. In particular, if a compound is optically active, the invention specifically includes the use of each of the compounds's enantiomers as well as racemic mixtures of the enantiomers.

In some embodiments, the topical formulations of the present invention are prepared using the free base form of the IRM compound.

In the present invention, the IRM is an imidazonaphthyridine amine. In other embodiments, the IRM is 2-methyl-1-(2-methylpropyl)-1*H*-imidazo[4,5-*c*][1,5]naphthyridin-4-amine.

The amount of an IRM compound that will be therapeutically effective in a specific situation will depend on such things as the activity of the particular compound, the dosing regimen, the application site, the particular formulation and the condition being treated. As such, it is generally not practical to identify specific administration amounts herein; however, those skilled in the art will be able to determine appropriate therapeutically effective amounts based on the guidance provided herein, information available in the art pertaining to these compounds, and routine testing. The term "a therapeutically effective amount" means an amount of the compound sufficient to induce a therapeutic or prophylactic effect, such as cytokine induction, inhibition of TH2 immune response, antiviral or antitumor activity, reduction or elimination of postsurgical scarring, reduction or resolution of actinic keratosis or pre-actinic keratosis lesions, reduction in the recurrence of actinic keratosis, or protection against uv-induced epidermal neoplasia, or as an adjuvant for therapeutic and prophylactic vaccines, including DNA, whole cell, protein subunit, attenuated virus, and all other vaccines, where the formulation may be applied before, during and/or after vaccine delivery.

In general, the amount of the IRM compound present in a topical formulation of the invention will be an amount effective to treat a targeted condition, to prevent recurrence of the condition, or to promote immunity against the condition. In certain embodiments, the amount or concentration of the IRM compound is at least 0.0001% by weight, such as, for example, at least 0.001%, at least 0.003%, at least 0.005%, at least 0.01%, at least 0.03%, at least 0.10%, at least 0.20%, at least 0.25%, at least 0.27%, at least 0.30%, and at least 1.0%, by weight based on the total weight of the formulation. In other embodiments, the amount of the IRM compound is at most 10% by weight, such as, for example, at most 5.0%, at most 3.0%, at most 1.0%, at most 0.5%, at most 0.4%, at most 0.35%, at most 0.33%, and at most 0.3%, by weight based on the total weight of the formulation.

### Preservative System

The formulation includes a preservative system. The preservative system includes one or more compounds that inhibit microbial growth (e.g., fungal and bacterial growth) within the formulation (for example, during manufacturing and use). The preservative system includes at least one preservative compound chosen from sorbic acid, esters or salts thereof, such as, for example, isopropyl sorbate, calcium sorbate, potassium sorbate, sodium sorbate, and triethanolammonium sorbate. Combinations of these may be used in formulations of the present invention. Such preservatives adversely affect the stability of the formulations as described herein.

According to the present invention, the sorbic acid preservative (i.e., sorbic acid, esters or salts thereof, or combinations thereof) is preferably present in a formulation in an amount of at least 0.005% by weight, more preferably at least 0.01% by weight, even more preferably at least 0.02% by weight, even more preferably at least 0.05% by weight, and even more preferably at least 0.08% by weight, based on the total weight of the formulation. The sorbic acid preservative is preferably present in a formulation in an amount of no greater than 1% by weight, more preferably no greater than 0.5% by weight, even more preferably no greater than 0.2% by weight, even more preferably no greater than 0.12% by weight, and even more preferably, no greater than 0.10% by weight, based on the total weight of the formulation.

In certain embodiments, in addition to the sorbic acid preservative, the preservative system will generally include at least one additional (i.e., secondary) preservative compound, such as, for example, methylparaben, ethylparaben, propylparaben, butylparaben, and phenoxyethanol. Various combinations of these compounds can be included in the preservative system. In some embodiments of the invention, the secondary preservative compound is methylparaben.

In some embodiments of the invention, the secondary preservative compound is present in an amount of at least 0.01% by weight, such as for example, at least 0.02%, at least 0.03%, at least 0.04%, and at least 0.05%, by weight based on the total weight of the formulation. In other embodiments of the invention the secondary preservative compound is present in an amount of at most 0.5 %, such as for example, at most 0.4%, at most 0.3%, and at most 0.2%, by weight based on the total weight of the formulation.

The preservative system may also include a preservative enhancing solubilizer which enhances the solubility of the preservative in the aqueous phase, examples of which include diethylene glycol monoethyl ether, propylene glycol, and poly(ethylene glycol)(4) monolaurate. Combinations of such enhancing solubilizers can be used in formulations of the present invention.

In some embodiments of the present invention, propylene glycol is present in an amount of at least 1.0% by weight, such as for example, at least 2.0%, at least 3.0%, at least 4.0%, and at least 5.0%, by weight based on the total weight of the formulation. In other embodiments of the present invention, propylene glycol is present in at most 10.0% by weight, such as for example, at most 8.0%, at most 6.0%, and at most 5.0%, by weight based on the total weight of the formulation.

### Antioxidants

Surprisingly, it has been discovered that the stability issue of the IRM/sorbic acid preservative combination can be addressed through the addition of one or more antioxidants. Antioxidants suitable for use herein are those that inhibit the autoxidation of the sorbic acid preservative. In particular, antioxidants having hydrogen atom donating functionality have demonstrated much greater improvement than others. Although not intending to be limiting, it is believed that antioxidants react with autoxidation intermediates (typically, radicals) of the sorbic acid preservative to form products that do not react with the IRM.

Suitable antioxidants are those that are pharmaceutically acceptable and described in the International Cosmetic Ingredient Dictionary and Handbook, Ninth Edition, Volume 4, 2002, and in the USP NF 2004: The United States Pharmacopeia, 27th Revision and The National Formulary, 22nd Edition.

Examples of suitable antioxidants include ascorbic acid (D and/or L enantiomers), ascorbyl palmitate (D and/or L enantiomers), butylated hydroxyanisole (BHA), butylated hydroxytoluene (BHT), cysteine (D and/or L enantiomers), propyl gallate, sodium formaldehyde sulfoxylate, sodium thiosulfate, sulfur dioxide, tocopherol, including all of its stereoisomers, and tocopherol polyethylene glycol 1000 succinate, including all of its stereoisomers.

Preferred antioxidants are those containing hydrogen atom donating functional groups. Examples of such antioxidants include ascorbic acid, ascorbyl palmitate, BHT, BHA, cysteine, propyl gallate, sodium formaldehyde sulfoxylate, tocopherol including all of its stereoisomers, and tocopherol polyethylene glycol 1000 succinate, including all of its stereoisomers.

More preferred antioxidants are those containing aromatic hydroxy groups capable of hydrogen atom donation. Examples of such antioxidants include BHA, BHT, propyl gallate, tocopherol, including all of its stereoisomers, and tocopherol polyethylene glycol 1000 succinate, including all of its stereoisomers.

Most preferred antioxidants are BHA and BHT, which can be used in combination.

According to the present invention, the antioxidant is preferably present in a formulation in an amount of at least 0.001% by weight, more preferably at least 0.005% by weight, even more preferably at least 0.008% by weight, and even more preferably at least 0.01% by weight, based on the total weight of the formulation. The antioxidant is preferably present in a formulation in an amount of no greater than 0.3% by weight, more preferably no greater than 0.2% by weight, and even more preferably no greater than 0.012% by weight, and even more preferably no greater than 0.1% by weight, based on the total weight of the formulation.

According to the present invention, the sorbic acid preservative (i.e., sorbic acid/ester/salt) to antioxidant weight ratio is preferably at least 1:20, more preferably at least 1:1, and even more preferably at least 5:1. The sorbic acid to antioxidant weight ratio is preferably no greater than 1000:1, more preferably no greater than 20:1, and even more preferably no greater than 10:1.

### Chelating Agents

In certain embodiments of the present invention, the formulation can also include at least one chelating agent. The chelating agent functions to stabilize the antioxidant(s) present in the formulation.

Chelating agents are compounds that complex with metal ions. Suitable chelating agents are those that are pharmaceutically acceptable and described in the International Cosmetic Ingredient Dictionary and Handbook, Ninth Edition, Volume 4, 2002.

Suitable chelating agents include ethylenediaminetetraacetic acid (EDTA) and citric acid, hydrates thereof, salts thereof, and hydrates of the salts thereof. Examples of such chelating agents include ethylenediaminetetraacetic acid disodium salt, ethylenediaminetetraacetic acid disodium salt dihydrate, and citric acid monohydrate. Various combinations of chelating agents can be used if desired.

According to the present invention, if included, the chelating agent is preferably present in a formulation in an amount of at least 0.001% by weight, more preferably at least 0.005% by weight, even more preferably at least 0.01% by weight, and even more preferably at least 0.05% by weight, based on the total weight of the formulation. The chelating agent is preferably present in a formulation in an amount of no greater than 0.2% by weight, and more preferably no greater than 0.1% by weight, based on the total weight of the formulation.

According to the present invention, if included, the antioxidant to chelating agent weight ratio is preferably at least 1:200, more preferably at least 1:10, and even more preferably at least 1:5. The antioxidant to chelating agent weight ratio is preferably no greater than 300:1, more preferably no greater than 10:1, and even more preferably no greater than 2:1.

### Fatty Acids

The topical formulations of the invention can additionally include a fatty acid. As used herein, the term "fatty acid" means a carboxylic acid, either saturated or unsaturated having 6 to 28 carbon atoms, such as, for example, from 10 to 22 carbon atoms. Non-limiting examples of such fatty acids include isostearic acid, oleic acid, and linear- or branched-chain carboxylic acids of 6 to 18 carbon atoms.

The fatty acid may be present in the formulation in an amount sufficient to solubilize the IRM compound. In certain embodiments, the amount of the fatty acid is at least 0.05% by weight, at least 1.0% by weight, at least 3.0% by weight, at least 5.0% by weight, at least 6.0% by weight, at least 7.0% by weight, at least 10% by weight, at least 15% by weight, or at least 25% by weight, based on the total weight of the formulation. In certain embodiments, the amount of the fatty acid is at most 40% by weight, at most 30% by weight, at most 15% by weight, at most 10% by weight, or at most 8.0% by weight based on the total weight of the formulation. The fatty acid component of the formulation can comprise one or more fatty acids.

### Hydrophobic Component

The topical formulations of the invention can additionally include at least one hydrophobic, aprotic component miscible with the fatty acid and comprising a hydrocarbyl group of 7 or more carbon atoms. By "hydrophobic" is meant that the component is essentially insoluble in water, i.e. immiscible with water and unable to form a micelle in water, and does not contain polyoxyethylene or acid salt groups. Preferably the hydrophobic, aprotic component has a hydrophilic lipophilic balance (HLB) of less than 2. The HLB of a component may be determined as described, for example, in Attwood, D., Florence, A. T. Surfactant Systems: Their Chemistry, Pharmacy, and Biology; New York: Chapman & Hall, 471-473, 1983. By "aprotic" is meant that the component cannot donate a proton to the IRM and does not contain groups such as carboxyl, hydroxy, primary and secondary amino, primary and secondary amido, or quaternary ammonium groups. Preferably this component has a pKa of at least 14.2 and does not substantially solubilize or form a complex such as an acid-base pair or complex or a hydrogen bond complex with the IRM compound. By "not substantially" is meant that the ratio of the IRM compound's solubility in the hydrophilic, aprotic component to that in isostearic acid is less than 1:40.

Formulations intended for dermal or topical use typically have amounts of an oil phase and a hydrophobic, aprotic component sufficient to provide desirable qualities such as spreadability and feel.

Examples of useful hydrophobic, aprotic components include but are not limited to fatty acid esters, for example, isopropyl mysristate, isopropyl palmitate, diisopropyl dimer dilinoleate; medium-chain (e.g., 8 to 14 carbon atoms) triglycerides, for example, caprylic/capric triglyceride; cetyl esters; hydrocarbons of 8 or more carbon atoms, for example, light mineral oil, white petrolatum; and waxes, for example, beeswax. In some embodiments, the hydrophobic, aprotic component is chosen from one or more of isopropyl mysristate, isopropyl palmitate, caprylic/capric triglyceride, and diisopropyl dimer dilinoleate. Various combinations of such hydrophobic, aprotic components can be used if desired.

In certain embodiments, the amount of the hydrophobic, aprotic component is at least 1.0% by weight, at least 3.0% by weight, at least 3.5% by weight, at least 4.0% by weight, at least 4.5% by weight, at least 5.0% by weight, or at least 10% by weight, based on the total weight of the formulation. In certain embodiments, the amount of the hydrophobic, aprotic component is at most 30% by weight, at most 15% by weight, at most 10% by weight, or at most 5.0% by weight based on the total weight of the formulation.

The weight ratio of the hydrophobic, aprotic component to the fatty acid can be 0.025:1 to 600:1, for example, 0.5:1 to 50:1, and 2:1 to 30:1. The combined amount (weight percent of the total topical formulation weight) of the hydrophobic, aprotic component and the fatty acid can be 2% to 50% by weight, for example 2% to 30%, 5% to 30%, 5% to 20%, and 10% to 20%.

### Viscosity Enhancing Agent

The formulations of the present invention can also comprise a viscosity enhancing agent. When water is the continuous phase, the viscosity enhancing agent will be a hydrophilic viscosity enhancing agent. Examples of suitable hydrophilic viscosity enhancing agents include cellulose ethers such as hydroxypropylmethylcellulose, hydroxyethylcellulose, hydroxypropylcellulose, and carboxymethylcellulose; polysaccharide gums such as xanthan gum; and homopolymers and copolymers of acrylic acid crosslinked with allyl sucrose or allyl pentaerythriol such as those polymers designated as carbomers in the United States Pharmacopoeia. Suitable carbomers include, for example, those available as CARBOPOL 934P, CARBOPOL 971P, CARBOPOL 940, CARBOPOL 974P, CARBOPOL 980, and PEMULEN TR-1 (USP/NF Monograph; Carbomer 1342), all available from Noveon, Cleveland, Ohio. In one embodiment of the present invention, the viscosity enhancing agent is chosen from CARBOPOL 974P and 980.

In certain embodiments, the amount of the viscosity enhancing agent, when used, is at least 0.1% by weight, at least 0.2% by weight, at least 0.5% by weight, at least 0.6% by weight, at least 0.7% by weight, at least 0.9% by weight, or at least 1.0% by weight, based on the total weight of the formulation. In certain embodiments, the amount of the viscosity enhancing agent, when used, is at most 10% by weight, at most 5.0% by weight, at most 3.0% by weight, at most 2.0% by weight, or at most 1.5% by weight, based on the total weight of the formulation.

### Emulsifier

The formulations of the invention can additionally comprise an emulsifier. Suitable emulsifiers include non-ionic surfactants such as, for example, polysorbate 60, sorbitan monostearate, polyglyceryl-4 oleate, polyoxyethylene(4) lauryl ether, etc. In certain embodiments, the emulsifier is chosen from poloxamers (e.g., PLURONIC F68, also known as POLOXAMER 188, a poly(ethylene glycol)-block-poly(propylene glycol)-block-poly(ethylene glycol), available from BASF, Ludwigshafen, Germany) and sorbitan trioleate (e.g., SPAN 85 available from Uniqema, New Castle, DE).

If included, the emulsifier is generally present in an amount of 0.1 % to 10% by weight of total formulation weight, for example, from 0.5% to 5.0% by weight, and from 0.75% to 3.5% by weight. In certain embodiments, the amount of the emulsifier, if used, is present in an amount of at least 0.1% by weight, at least 0.5% by weight, at least 0.75% by weight, at least 1.0% by weight, at least 2.5% by weight, at least 3.5% by weight, or at least 5.0% by weight, based on the total weight of the formulation. In certain embodiments, the amount of the emulsifier, if used, is present in an amount of at most 10% by weight, at most 5.0% by weight, or at most 3.5% by weight, based on the total weight of the formulation.

### pH Adjuster

The formulations of the present invention may additionally include at least one pH adjuster. Suitable pH adjusters include organic bases and inorganic bases such as, for example, KOH and NaOH (e.g., aqueous formulations). The pH of the topical formulations of the present invention generally ranges from 3.5 to 7.0. In one embodiment, the pH of the topical formulations of the present invention can range from 4.0 to 6.0, preferably 5.0.

### Illustrative Formulations

Preferred formulations of the present invention are as follows. The water used is typically purified water.

In one embodiment of the present invention, a topical formulation includes:
0.001% by weight to 5.0% by weight of an imidazonaphthyridine amine (preferably, 2-methyl-1-(2-methylpropyl)-1*H*-imidazo[4,5-*c*][1,5]naphthyridin-4-amine);
0.02% by weight to 0.2% by weight of a sorbic acid preservative selected from the group consisting of sorbic acid, esters thereof, salts thereof, and combinations thereof;
0 to 10.0% by weight of propylene glycol;
0.05% by weight to 0.2% by weight of methylparaben;
0.001% by weight to 0.2% by weight of butylated hydroxyanisole, butylated hydroxytoluene, or combinations thereof;
0 to 0.1% by weight of ethylenediaminetetraacetic acid, a hydrate thereof, a salt thereof, a hydrate of a the salt thereof, or combinations thereof;
1% buy weight to 30% by weight of isostearic acid;
1% by weight to 15% by weight of a medium-chain triglyceride;
0.2% by weight to 2.0% by weight of a carbomer;
0.1 % by weight to 6.0% by weight of a poloxamer; and water;
wherein the formulation has a pH of 4.0 to 6.0 and the weight percentages are based on the total weight of the formulation.

In one embodiment, a topical formulation includes:
0.3% by weight of 2-mothyl-1-(2-methylpropyl)-1*H*-imidazo[4,5-*c*][1,5]naphthyridin-4-amine;
0.15% by weight sorbic acid;
5.0% by weight propylene glycol;
0.2% by weight methylparaben;
0.1% by weight butylated hydroxyanisole;
0.05% by weight ethylenediaminetetraacetic acid disodium salt dihydrate;
7.0% by weight isostearic acid;
4.0% by weight of caprylic/capric triglyceride;
1.0% by weight of a carbomer;
3.5% by weight of a poloxamer;
0.8% by weight of an aqueous solution of 20% by weight NaOH in water; and
77.9% by weight water;
wherein the weight percentages are based on the total weight of the formulation.

In one embodiment, a topical formulation includes:
0.30% by weight of 2-methyl-1-(2-methylpropyl)-1*H*-imidazo[4,5-*c*][1,5]naphthyridin-4-amine;
0.10% by weight sorbic acid;
5.00% by weight propylene glycol;
0.20% by weight methylparaben;
0.01% by weight butylated hydroxyanisole;
0.05% by weight ethylenediaminetetraacetic acid disodium salt dihydrate;
7.00% by weight isostearic acid;
4.00% by weight of caprylic/capric triglyceride;
1.00% by weight of a carbomer;
3.50% by weight of a poloxamer;
0.80% by weight of an aqueous solution of 20% by weight NaOH in water; and
78.04% by weight water;
wherein the weight percentages are based on the total weight of the formulation.

In one embodiment, a topical formulation includes:
0.3% by weight of 2-methyl-1-(2-methylpropyl)-1*H-*imidazo[4,5-*c*][1,5]naphthyridin-4-amine;
0.1 % by weight sorbic acid;
5.0% by weight propylene glycol;
0.2% by weight methylparaben;
0.01 % by weight butylated hydroxyanisole;
0.05% by weight ethylenediaminetetraacetic acid disodium salt dihydrate;
7.0% by wight isostearic acid;
4.0% by weight of caprylic/capric triglyceride;
1.0% by weight of a carbomer;
3.5% by weight of a poloxamer;
0.8% by weight of an aqueous solution of 20% by weight NaOH in water; and
78.0% by weight water;
wherein the weight percentages are based on the total weight of the formulation.

In one embodiment, a topical formulation includes:
0.03% by weight of 2-methyl-1-(2-methylpropyl)-1*H*-imidazo[4,5-*c*][1,5]naphthyridin-4-amine;
0.15% by weight sorbic acid;
5.0% by weight propylene glycol;
0.2% by weight methylparaben;
0.1% by weight butylated hydroxyanisole;
0.05% by weight ethylenediaminetetraacetic acid disodium salt dihydrate;
5.0% by weight isostearic acid;
4.0% by weight of caprylic/capric triglyceride;
1.0% by weight of a carbomer;
3.5% by weight of a poloxamer;
0.8% by weight of an aqueous solution of 20% by weight NaOH in water; and
80.17% by weight water;
wherein the weight percentages are based on the total weight of the formulation.

In one embodiment, a topical formulation includes:
0.1% by weight of 2-methyl-1-(2-methylpropyl)-1*H*-imidazo[4,5-*c*][1,5]naphthyridin-4-amine;
0.15% by weight sorbic acid;
5.0% by weight propylene glycol;
0.2% by weight methylparaben;
0.1 % by weight butylated hydroxyanisole;
0.05% by weight ethylenediaminetetraacetic acid disodium salt dihydrate;
5.0% by weight isostearic acid;
4.0% by weight of caprylic/capric triglyceride;
1.0% by weight of a carbomer;
3.5% by weight of a poloxamer;
0.8% by weight of an aqueous solution or 20% by weight NaOH in water; and
80.1 % by weight water;
wherein the weight percentages are based on the total weight of the formulation.

### Methods of Application

Formulations according to the present invention can be applied to any suitable location, for example topically to dermal and/or mucosal surfaces, or internally to a particular tissue location. In the case of dermal application, for example, depending on the IRM compound concentration, formulation composition, and dermal surface, the therapeutic effect of the IRM compound may extend only to the superficial layers of the dermal surface or to tissues below the dermal surface. Thus, another aspect of the present invention is directed to a method for the treatment of a dermal and/or mucosal associated condition comprising applying to skin one of the foregoing formulations. As used herein, a "dermal and/or mucosal associated condition" means an inflammatory, infectious, neoplastic or other condition that involves a dermal and/or mucosal surface or that is in sufficient proximity to a dermal and/or mucosal surface to be affected by a therapeutic agent topically applied to the surface. Examples of a dermal and/or mucosal associated condition include warts, atopic dermatitis, postsurgical scars, lesions caused by a herpes virus, and epidermal neoplasias, such as for example actinic keratosis, pre-actinic keratosis lesions, malignant melanomas, basal cell carcinoma, and squamous cell carcinoma.

In one embodiment, the formulations can be applied to the surface of skin for treatment of actinic keratosis (AK). Actinic keratoses are premalignant lesions considered biologically to be either carcinoma in-situ or squamous intraepidermal neoplasia. AK is the most frequent epidermal tumor and is induced by ultraviolet (UV) radiation, typically from sunlight. Because of its precancerous nature, AK may be considered the most important manifestation of sun-induced skin damage.

In some embodiments, the above described formulations are particularly advantageous for dermal and/or mucosal application for a period of time sufficient to obtain a desired therapeutic effect without undesired systemic absorption of the IRM.

The precise amount of formulation effective for treating a dermal and/or mucosal associated condition will vary according to factors known in the art including but not limited to the particular IRM compound, the particular formulation, the intended dosing regimen, the particular condition being treated, the state of the subject's immune system (e.g., suppressed, compromised, stimulated), and the species to which the formulation is being administered. In some embodiments the amount of formulation is an amount sufficient to deliver a dose of about 0.02 mg to about 15 mg of IRM compound. In other embodiments the amount of formulation is an amount sufficient to deliver a dose of about 0.2 mg to about 2.5 mg of IRM compound. In other embodiments the amount of formulation is an amount sufficient to deliver a dose of about 0.5 mg to about 1.7 mg of IRM compound. In one particular embodiment a dose of 0.75 mg of IRM compound is delivered. In another particular embodiment a dose of 1.5 mg of IRM compound is delivered.

The dosing regimen will vary at least in part on many factors known in the art including but not limited to the particular IRM compound, the particular formulation, the amount of formulation being administered, the particular condition being treated, the state of the subject's immune system (e.g., suppressed, compromised, stimulated), and the species to which the formulation is being administered. In some embodiments the formulation is administered at least once a week, at least twice a week, or at least three times a week. In other embodiments the formulation is administered at most seven times a week, at most six times a week, at most five times a week, or at most four times a week. In some embodiments the formulation is administered for at least two weeks, for at least four weeks, for at least six weeks, or for at least eight weeks. In other embodiments the formulation is administered for at most sixteen weeks, for at most twelve weeks, or for at most eight weeks. In some embodiments, about 200 to about 600 mg of formulation is administered twice a week for eight weeks. In one particular embodiment, about 250 mg of the formulation described in Example 22 below is administered twice a week for eight weeks. In another particular embodiment, about 500 mg of the formulation described in Example 22 below is administered twice a week for eight weeks.

### EXAMPLES

The following Examples are provided to further describe various IRM formulations and methods according to the invention. The examples, however, are not intended to limit the formulations and methods within the scope of the invention.

### TEST METHODS

### Test Method 1 - IRM 1 Compound Content and Sorbic Acid Content

A gradient reversed phase high performance liquid chromatography (HPLC) method was used to determine the amount of 2-methyl-1-(2-methylpropyl)-1*H-*imidazo[4,5-*c*][1,5]naphthyridin-4-amine (IRM Compound 1) and sorbic acid in cream formulations.

HPLC parameters: Analytical column: ZORBAX RX-C8, 5.0 micron particle, 150 x 4.6 mm (available from Agilent Technologies, Wilmington, Delaware, USA); Column temperature: 30°C; Detector: UV at 254 nm; Flow Rate: 1.0 mL/min; Injection volume: 25 µL; Mobile phase A: 62% aqueous (0.2% sodium 1-octanesulfonate, 0.2% triethylamine, 0.2% of 85% phosphoric acid), 21% acetonitrile, 17% methanol; Mobile Phase B: 20% aqueous (0.2% sodium 1-octanesulfonate, 0.2% triethylamine, 0.2% of 85% phosphoric acid), 42% acetonitrile, 38% methanol; Data acquisition time: 23 minutes; HPLC run time: approximately 30 minutes.

Gradient program: 0 minutes: 100% mobile phase A, 0% mobile phase B; 2.5 minutes: 100% mobile phase A, 0% mobile phase B; 10 minutes: 49% mobile phase A, 51 % mobile phase B; 14 minutes: 49% mobile phase A, 51 % mobile phase B; 20 minutes: 0% mobile phase A, 100% mobile phase B; 23 minutes: 0% mobile phase A, 100% mobile phase B; 25 minutes: 100% mobile phase A, 0% mobile phase B; 30 minutes: 100% mobile phase A, 0% mobile phase B.

IRM Compound 1 sample solution: A portion of the cream formulation (1000 mg for creams containing 0.01, 0.03, 0.05 and 0.1% IRM and 250 mg for creams containing 0.3, 0.6, and 1.0% IRM) was accurately weighed into a volumetric flask (50 mL for the 1000 mg samples and 100 mL for the 250 mg samples). Approximately 40 mL of diluent (prepared by combing 200 parts of acetonitrile, 790 parts water, and 10 parts phosphoric acid, all parts by volume) was added to the 50 mL flask or 80 mL to the 100 mL flask. The flask was sonicated with occasional shaking for 20 minutes or until the cream was completely dispersed. The solution was allowed to cool to ambient temperature and then diluted to volume with diluent. A portion of the solution was filtered using a syringe equipped with a 0.2 micron PTFE filter to provide the sample solution.

Sorbic acid sample solution: A 250 mg portion of cream was accurately weighed into a 100 mL volumetric flask. Approximately 80 mL of diluent (prepared by combing 200 parts of acetonitrile, 790 parts water, and 10 parts phosphoric acid, all parts by volume) was added to the flask. The flask was sonicated with occasional shaking for 20 minutes or until the cream was completely dispersed. The solution was allowed to cool to ambient temperature and then diluted to volume with diluent. A portion of the solution was filtered using a syringe equipped with a 0.2 micron PTFE filter to provide the sample solution.

### Test Method 2 - BHA Content

A gradient reversed phase high performance liquid chromatography (HPLC) method was used to determine the amount of BHA in cream formulations containing IRM Compound 1.

HPLC parameters: Analytical column: ZORBAX Bonus RP, 3.5 micron particle, 150 x 3.0 mm; Column temperature: 40°C; Detector: UV at 290 nm; Flow Rate: 0.5 mL/min; Injection volume: 20 µL; Mobile phase A: 0.1% formic acid in water; Mobile Phase B: 0.05% formic acid in acetonitrile; Data acquisition time: 12 minutes; HPLC run time: approximately 20 minutes.

Gradient program: 0 minutes: 60% mobile phase A, 40% mobile phase B; 10 minutes: 5% mobile phase A, 95% mobile phase B; 12 minutes: 5% mobile phase A, 95% mobile phase B; 13 minutes: 60% mobile phase A, 40% mobile phase B; 20 minutes: 60% mobile phase A, 40% mobile phase B.

Sample solution: A portion (approximately 1000 mg) of the cream formulation was accurately weighed into a 100 mL volumetric flask. Approximately 80 mL of diluent (prepared by combining 600 parts of acetonitrile, 400 parts of water, and 1 part formic acid, all parts by volume) was added and the flask was sonicated with occasional shaking for 10 minutes or until the cream was well dispersed. The solution was allowed to cool to ambient temperature and then diluted to volume with diluent. A portion of the solution was filtered using a syringe equipped with a 0.2 micron PTFE filter to provide the sample solution.

### Test Method 3 - IRM Compound 1 Content

A gradient reversed phase high performance liquid chromatography (HPLC) method was used to determine the amount of 2-methyl-1-(2-methylpropyl)-1*H-*imidazo[4,5-*c*][1,5]naphthyridin-4-amine (IRM Compound 1) in cream formulations using BHA and BHT as the antioxidants.

HPLC parameters: Analytical column: ZORBAX Bonus RP, 3.5 micron particle, 150 x 4.6 mm (available from Agilent Technologies, Wilmington, Delaware, USA); Column temperature: 35°C; Detector: UV at 240 nm; Flow Rate: 1.0 mL/min; Injection volume: 30 µL; Mobile phase A: 0.05% trifluoroacetic acid in water; Mobile Phase B: 0.05% trifluoroacetic acid in acetonitrile; Data acquisition time: 25 minutes; HPLC run time: 35 minutes.

Gradient program: 0 minutes: 80% mobile phase A, 20% mobile phase B; 5 minutes: 80% mobile phase A, 20% mobile phase B; 15 minutes: 75% mobile phase A, 25% mobile phase B; 25 minutes: 35% mobile phase A, 65% mobile phase B; 28 minutes: 10% mobile phase A, 90% mobile phase B; 29 minutes: 80% mobile phase A, 20% mobile phase B; 35 minutes: 80% mobile phase A, 20% mobile phase B.

Sample solution: A portion of the cream formulation (2500 mg for creams containing 0.03% IRM; 1500 mg for creams containing 0.1% IRM; and 500 mg for creams containing 0.3% IRM) was accurately weighed into a volumetric flask (50 mL for creams containing 0.03% IRM; 100 mL for creams containing 0.1 or 0.3% IRM). Approximately 40 mL of diluent (prepared by combing 200 parts of acetonitrile, 790 parts water, and 10 parts phosphoric acid, all parts by volume) was added to the 50 mL flask or 80 mL to the 100 mL flask. The flask was shaken or vortexed to dislodge any cream from the neck of the flask and then sonicated with occasional shaking for 10 minutes or until the cream was completely dispersed. The solution was allowed to cool to ambient temperature and then diluted to volume with diluent. A portion of the solution was filtered using a syringe equipped with a 0.2 micron PTFE filter to provide the sample solution.

### Test Method 4 - Sorbic Acid and BHA Content

A gradient reversed phase high performance liquid chromatography (HPLC) method was used to determine the amount of sorbic acid and BHA in cream formulations containing IRM Compound 1.

HPLC parameters: Analytical column: ZORBAX Bonus RP, 3.5 micron particle, 150 x 4.6 mm; Column temperature: 35°C; Detector: UV at 285 nm; Flow Rate: 1.0 mL/min; Injection volume: 25 µL; Mobile phase A: 0.05% trifluoroacetic acid in water; Mobile Phase B: 0.05% trifluoroacetic acid in acetonitrile; Data acquisition time: 12 minutes; HPLC run time: 18 minutes.

Gradient program: 0 minutes: 60% mobile phase A, 40% mobile phase B; 10 minutes: 5% mobile phase A, 95% mobile phase B; 12 minutes: 5% mobile phase A, 95% mobile phase B; 13 minutes: 60% mobile phase A, 40% mobile phase B; 18 minutes: 60% mobile phase A, 40% mobile phase B.

Sample solution: A portion (approximately 1000 mg) of the cream formulation was accurately weighed into a 100 mL volumetric flask. Approximately 80 mL of diluent (prepared by combining 600 parts of acetonitrile, 400 parts of water, and 1 part trifluoroacetic acid, all parts by volume) was added and the flask was sonicated with occasional shaking for 10 minutes or until the cream was well dispersed. The solution was allowed to cool to ambient temperature and then diluted to volume with diluent. A portion of the solution was filtered using a syringe equipped with a 0.45 micron PTFE filter to provide the sample solution.

### Preparation of Cream Formulations

The cream formulations in the Examples below were prepared using the following general method.

Oil phase preparation: The IRM compound and the BHA or BHT were dissolved in the isostearic acid and medium chain triglycerides, with heat if necessary. Generally the CARBOPOL 980 was then dispersed in the oil phase.

Water phase preparation: Edetate disodium dihydrate, methylparaben, sorbic acid, propylene glycol, and POLOXAMER 188 were added to the water and mixed until dissolved, with heat if necessary. If the CARBOPOL was not dispersed in the oil phase, it was dispersed in the water phase.

Phase combination: The oil phase was added to the water phase at ambient conditions. The emulsion was then homogenized. Sodium hydroxide was added either before or after phase combination. The cream was mixed until smooth and uniform. The pH of the cream was measured and a pH adjustment was made with additional sodium hydroxide solution, if necessary, to meet the in-process target of pH 5.

### Examples 1-6

Table 1 summarizes topical formulations made in accordance with the present invention in a percentage weight-by-weight basis. The formulations were packaged in aluminum tubes with an epoxy phenolic lacquer liner.

| Table 1 | | | | | | |
|---|---|---|---|---|---|---|
| Ingredient | Ex 1 | Ex 2 | Ex 3 | Ex 4 | Ex 5 | Ex6 |
| IRM 1 | 0.01 | 0.03 | 0.10 | 0.30 | 0.60 | 1.00 |
| Isostearic acid | 5.00 | 5.00 | 5.00 | 7.00 | 10.00 | 10.00 |
| *Medium-chain Triglycerides | 4.00 | 4.00 | 4.00 | 4.00 | 4.00 | 4.00 |
| CARBOPOL 980 | 1.00 | 1.00 | 1.00 | 1.00 | 1.00 | 1.00 |
| POLOXAMER 188 | 3.50 | 3.50 | 3.50 | 3.50 | 3.50 | 3.50 |
| Propylene glycol | 5.00 | 5.00 | 5.00 | 5.00 | 5.00 | 5.00 |
| Methylparaben | 0.20 | 0.20 | 0.20 | 0.20 | 0.20 | 0.20 |
| Sorbic acid | 0.15 | 0.15 | 0.15 | 0.15 | 0.15 | 0.15 |
| BHA | 0.10 | 0.10 | 0.10 | 0.10 | 0.10 | 0.10 |
| Edetate disodium dihydrate | 0.05 | 0.05 | 0.05 | 0.05 | 0.05 | 0.05 |
| Sodium hydroxide Solution 20% w/w | 0.80 | 0.80 | 0.80 | 0.80 | 0.80 | 0.80 |
| Purified water | 80.19 | 80.17 | 80.10 | 77.90 | 74.60 | 74.20 |

| | | | | | | |
|---|---|---|---|---|---|---|
| *Caprylic/capric triglyceride available under the trade names CRODAMOL GTCC-PN (Croda, Inc) and MIGLYOL 812N (Sasol). | | | | | | |

The creams of Examples 1-6 were stored at 40°C at 75% relative humidity. At selected time points samples were analyzed for IRM 1, sorbic acid (SA), and BHA content. The results are shown in Table 2 below. The initial values (0 month) are the average of 6 independent determinations (2 samples from each of 3 tubes); the values for the later time points are the average of 2 independent determinations (2 samples from 1 tube). Values are not normalized for weight loss that may have occurred during storage. Test Method 1 was used to determine the IRM 1 content and sorbic acid content. Test Method 2 was used to determine the BHA content.

| Table 2 | | | | | | |
|---|---|---|---|---|---|---|
| Ingredient and Time point (40°C/75%RH) | Content (% w/w) | | | | | |
| | (% Initial) | | | | | |
| | Ex 1 | Ex 2 | Ex 3 | Ex 4 | Ex 5 | Ex 6 |
| IRM 1 - 0 month | 0.01004 (100.0) | 0.0302 (100.0) | 0.1001 (100.0) | 0.306 (100.0) | 0.609 (100.0) | 1.008 (100.0) |
| IRM 1 - 1 month | 0.01009 (100.5) | 0.0302 (100.0) | 0.1004 (100.3) | 0.306 (100.0) | 0.612 (100.5) | 1.017 (100.9) |
| IRM 1 - 2 month | 0.00990 (98.6) | 0.0301 (99.7) | 0.0999 (99.8) | 0.308 (100.7) | 0.602 (98.9) | 0.993 (98.5) |
| IRM 1 - 3 month | 0.01012 (100.8) | 0.0297 (98.3) | 0.0985 (98.4) | 0.301 (98.4) | 0.615 (101.0) | 1.026 (101.8) |
| IRM 1 - 6 month | 0.01008 (100.4) | 0.0301 (99.7) | 0.1000 (99.9) | 0.307 (100.3) | 0.616 (101.1) | 1.025 (101.7) |
| SA - 0 month | 0.152 (100.0) | 0.155 (100.0) | 0.152 (100.0) | 0.149 (100.0) | 0.150 (100.0) | 0.150 (100.0) |
| SA - 1 month | 0.152 (100.0) | 0.153 (98.7) | 0.152 (100.0) | 0.148 (99.3) | 0.150 (100.0) | 0.150 (100.0) |
| SA - 2 month | 0.155 (102.0) | 0.152 (98.1) | 0.151 (99.3) | 0.149 (100.0) | 0.149 (99.3) | 0.148 (98.7) |
| SA - 3 month | 0.152 (100.0) | 0.151 (97.4) | 0.149 (98.0) | 0.147 (98.7) | 0.147 (98.0) | 0.148 (98.7) |
| SA - 6 month | 0.150 (98.7) | 0.153 (98.7) | 0.153 (100.7) | 0.148 (99.3) | 0.150 (100.0) | 0.150 (100.0) |
| BHA - 0 month | 0.1029 (100.0) | 0.1057 (100.0) | 0.1086 (100.0) | 0.1030 (100.0) | 0.1025 (100.0) | 0.1030 (100.0) |
| BHA - 1 month | 0.1049 (101.9) | 0.1017 (96.2) | 0.1019 (93.8) | 0.1005 (97.6) | 0.1019 (99.4) | 0.1014 (98.4) |
| BHA - 2 month | 0.0995 (96.7) | 0.1026 (97.1) | 0.1029 (94.8) | 0.1002 (97.3) | 0.1001 (97.7) | 0.0975 (94.7) |
| BHA - 3 month | 0.0978 (95.0) | 0.1011 (95.6) | 0.0984 (90.6) | 0.0978 (95.0) | 0.0984 (96.0) | 0.0973 (94.5) |
| BHA - 6 month | 0.1029 (100.0) | 0.1004 (95.0) | 0.1007 (92.7) | 0.1001 (97.2) | 0.1008 (98.3) | 0.1018 (98.8) |

### Examples 7 & 8

Table 3 summarizes topical formulations made in accordance with the present invention in a percentage weight-by-weight basis and a formulation prepared without an antioxidant (C1). The formulations were packaged in glass containers.

| Table 3 | | | |
|---|---|---|---|
| Ingredient | Ex 7 | Ex 8 | Ex C1 |
| IRM 1 | 0.30 | 0.30 | 0.30 |
| Isostearic acid | 7.00 | 7.00 | 7.00 |
| *Medium-chain Triglycerides | 8.00 | 8.00 | 8.00 |
| CARBOPOL 980 | 1.00 | 1.00 | 1.00 |
| POLOXAMER 188 | 2.50 | 2.50 | 2.50 |
| Propylene glycol | 5.00 | 5.00 | 5.00 |
| Methylparaben | 0.20 | 0.20 | 0.20 |
| Sorbic acid | 0.15 | 0.15 | 0.15 |
| BHA | 0.10 | - | - |
| BHT | - | 0.10 | - |
| Edetate disodium dihydrate | 0.05 | 0.05 | 0.05 |
| Sodium hydroxide Solution 20% w/w | 0.80 | 0.80 | 0.80 |
| Purified water | 74.90 | 74.90 | 75.00 |

| | | | |
|---|---|---|---|
| *Caprylic/capric triglyceride available under the trade names CRODAMOL GTCC-PN (Croda, Inc) and MIGLYOL 812N (Sasol). | | | |

The creams of Examples 7, 8, and C1 where stored at 40°C at 75% relative humidity and at 55°C at ambient humidity. At selected time points samples were analyzed using Test Method 1 described above for IRM 1 and sorbic acid content. The results are shown in Table 4 below where each value is for 1 sample from 1 container of cream. Values were not normalized for weight loss that may have occurred during storage.

| Table 4 | | | |
|---|---|---|---|
| Ingredient - Time point (Conditions) | Content (% w/w) | | |
| | (% Initial) | | |
| | Ex 7 | Ex 8 | Ex C1 |
| IRM 1 - 0 month (40°C) | 0.303 (100.0) | 0.303 (100.0) | 0.304 (100.0) |
| IRM 1 - 1 month (40°C) | 0.302 (99.7) | 0.306 (101.0) | 0.302 (99.3) |
| IRM 1 - 2 month (40°C) | 0.305 (100.7) | 0.308 (101.7) | 0.307 (101.0) |
| IRM 1 - 3 month (40°C) | 0.308 (101.7) | 0.315 (104.0) | 0.303 (99.7) |
| IRM 1 - 6 month (40°C) | 0.305 (100.7) | 0.313 (103.3) | 0.296 (97.4) |
| SA - 0 month (40°C) | 0.147 (100.0) | 0.147 (100.0) | 0.147 (100.0) |
| SA - 1 month (40°C) | 0.146 (99.3) | 0.147 (100.0) | 0.140 (95.2) |
| SA - 2 month (40°C) | 0.145 (98.6) | 0.147 (100.0) | 0.133 (90.5) |
| SA - 3 month (40°C) | 0.147 (100.0) | 0.150 (102.0) | 0.126 (85.7) |
| SA - 6 month (40°C) | 0.146 (99.3) | 0.148 (100.7) | 0.119 (81.0) |
| IRM 1 - 0 weeks (55°C) | 0.303 (100.0) | 0.303 (100.0) | 0.304 (100.0) |
| IRM 1 - 2 weeks (55°C) | 0.302 (99.7) | 0.304 (100.3) | 0.301 (99.0) |
| IRM 1 - 4 weeks (55°C) | 0.303 (100.0) | 0.308 (101.7) | 0.301 (99.0) |
| IRM 1 - 6 weeks (55°C) | 0.307 (101.3) | 0.311 (102.6) | 0.301 (99.0) |
| IRM 1 - 8 weeks (55°C) | 0.311 (102.6) | 0.314 (103.6) | 0.298 (98.0) |
| SA - 0 weeks (55°C) | 0.147 (100.0) | 0.147 (100.0) | 0.147 (100.0) |
| SA - 2 weeks (55°C) | 0.146 (99.3) | 0.147 (100.0) | 0.138 (93.9) |
| SA - 4 weeks (55°C) | 0.146 (99.3) | 0.148 (100.7) | 0.133 (90.5) |
| SA - 6 weeks (55°C) | 0.148 (100.7) | 0.148 (100.7) | 0.125 (85.0) |
| SA - 8 weeks (55°C) | 0.148 (100.7) | 0.149 (101.4) | 0.118 (80.3) |

### Examples 9-18

Table 5 summarizes topical formulations made in accordance with the present invention in a percentage weight-by-weight basis. The formulations were packaged in aluminum tubes with an epoxy phenolic lacquer liner. The formulations of Examples 9-18 were stored at 40°C at 75% relative humidity. At selected time points samples were analyzed for IRM 1, sorbic acid (SA), and BHA content. The results are shown in Table 6 below where the initial values of IRM and SA are the average of 6 independent determinations (2 samples from each of 3 tubes), the initial BHA values are the average of 3 independent determinations (1 sample from each of 3 tubes), and the values for later time points are the values for one sample from 1 tube. Values are not normalized for weight loss that may have occurred during storage. Test Method 1 was used to determine the IRM 1 content and sorbic acid content. Test Method 2 was used to determine the BHA content.

| Table 5 | | | | | | | | | | |
|---|---|---|---|---|---|---|---|---|---|---|
| Ingredient | Ex 9 | Ex 10 | Ex 11 | Ex 12 | Ex 13 | Ex 14 | Ex 15 | Ex 16 | Ex 17 | Ex 18 |
| IRM1 | 0.30 | 0.30 | 0.30 | 0.30 | 0.30 | 0.30 | 0.30 | 0.30 | 0.30 | 0.30 |
| Isostearic acid | 7.00 | 7.00 | 7.00 | 7.00 | 7.00 | 7.00 | 7.00 | 7.00 | 7.00 | 7.00 |
| *Medium-chain Triglycerides | 4.00 | 4.00 | 4.00 | 4.00 | 4.00 | 4.00 | 4.00 | 4.00 | 4.00 | 4.00 |
| CARBOPOL 980 | 1.00 | 1.00 | 1.00 | 1.00 | 1.00 | 1.00 | 1.00 | 1.00 | 1.00 | 1.00 |
| Poloxamer 188 | 3.50 | 3.50 | 3.50 | 3.50 | 3.50 | 3.50 | 3.50 | 3.50 | 3.50 | 3.50 |
| Propylene glycol | *5.00* | *5.00* | *5.00* | *5.00* | *5.00* | *5.00* | *5.00* | *5.00* | *5.00* | *5.00* |
| Methylparaben | 0.20 | 0.20 | 0.20 | 0.20 | 0.20 | 0.20 | 0.20 | 0.20 | 0.20 | 0.20 |
| Sorbic acid | 0.01 | 0.10 | 0.06 | 0.10 | 0.06 | 0.10 | 0.08 | 0.08 | 0.08 | 0.08 |
| BHA | 0.06 | 0.10 | 0.006 | 0.01 | 0.011 | 0.018 | 0.08 | 0.008 | 0.015 | 0.015 |
| Edetate disodium dihydrate | - | - | - | - | - | - | - | - | - | 0.05 |
| Sodium hydroxide Solution 20% w/w | ** | ** | ** | ** | ** | ** | ** | ** | ** | ** |
| Purified water | *** | *** | *** | *** | *** | *** | *** | *** | *** | *** |

| | | | | | | | | | | |
|---|---|---|---|---|---|---|---|---|---|---|
| *Caprylic/capric triglyceride available under the trade names CRODAMOL GTCC-PN (Croda, Inc) and MIGLYOL 812N (Sasol). ** *qs to* pH *5* ***qs to 100 | | | | | | | | | | |

| Table 6 | | | | | | | | | | |
|---|---|---|---|---|---|---|---|---|---|---|
| Ingredient - Timepoint | Content (% w/w) | | | | | | | | | |
| | (% Initial) | | | | | | | | | |
| | Ex 9 | Ex 10 | Ex 11 | Ex 12 | Ex 13 | Ex 14 | Ex 15 | Ex 16 | Ex 17 | Ex 18 |
| IRM 1 - 0 month | 0.312 (100.0) | 0.311 (100.0) | 0.307 (100.0) | 0.308 (100.0) | 0.309 (100.0) | 0.310 (100.0) | 0.308 (100.0) | 0.307 (100.0) | 0.309 (100.0) | 0.309 (100.0) |
| IRM 1 - 2 month | 0.310 (99.4) | 0.310 (99.7) | 0.306 (99.7) | 0.308 (100.0) | 0.311 (100.6) | 0.309 (99.7) | 0.304 (98.7) | 0.307 (100.0) | 0.308 (99.7) | 0.303 (98.1) |
| IRM 1 - 4 month | 0.313 (100.3) | 0.310 (99.7) | 0.313 (102.0) | 0.308 (100.0) | 0.311 (100.6) | 0.306 (98.7) | 0.304 (98.7) | 0.311 (101.3) | 0.310 (100.2) | 0.310 (100.3) |
| IRM 1 - 6 month | 0.314 (100.6 | 0.310 (99.7) | 0.311 (101.3) | 0.309 (100.3) | 0.314 (101.6) | 0.319 (102.9) | 0.311 (101.0) | 0.313 (102.0) | 0.313 (101.3) | 0.316 (102.3) |
| SA - 0 month | 0.0598 (100.0) | 0.1012 (100.0) | 0.0600 (100.0) | 0.1006 (100.0) | 0.0608 (100.0) | 0.1014 (100.0) | 0.0808 (100.0) | 0.0803 (100.0) | 0.0809 (100.0) | 0.0807 (100.0) |
| SA - 2 month | 0.0580 (97.0) | 0.0986 (97.4) | 0.0586 (97.7) | 0.0987 (98.1) | 0.0596 (98.0) | 0.0995 (98.1) | 0.0784 (97.0) | 0.0789 (98.3) | 0.0794 (98.1) | 0.0788 (97.6) |
| SA - 4 month | 0.0571 (95.5) | 0.0962 (95.1) | 0.0582 (97.0) | 0.0956 (95.0) | 0.0585 (96.2) | 0.0968 (95.5) | 0.0767 (94.9) | 0.0773 (96.3) | 0.0779 (96.3) | 0.0792 (98.1) |
| SA - 6 month | 0.0565 (94.5) | 0.0951 (94.0) | 0.0578 (96.3) | 0.0957 (95.1) | 0.0590 (97.0) | 0.0998 (98.4) | 0.0773 (95.7) | 0.0780 (97.1) | 0.0776 (95.9) | 0.0812 (100.6) |
| BHA - 0 month | 0.0610 (100.0) | 0.1016 (100.0) | 0.0058 (100.0) | 0.0100 (100.0) | 0.0110 (100.0) | 0.0180 (100.0) | 0.0935 (100.0) | 0.0078 (100.0) | 0.0150 (100.0) | 0.0151 (100.0) |
| BHA - 2 month | 0.0532 (87.2) | 0.0865 (85.1) | 0.0043 (73.9) | 0.0078 (78.3) | 0.0090 (81.7) | 0.0148 (82.5) | 0.0850 (90.9) | 0.0063 (81.1) | 0.0119 (79.3) | 0.0156 (103.1) |
| BHA - 4 month | 0.0430 (70.5) | 0.0717 (70.6) | 0.0036 (62.4) | 0.0061 (60.9) | 0.0077 (70.1) | 0.0125 (69.7) | 0.0706 (75.5) | 0.0054 (69.5) | 0.0099 (66.0) | 0.0146 (96.8) |
| BHA - 6 month | 0.0388 (63.6) | 0.0690 (67.9) | 0.0030 (51.7) | 0.0055 (55.0) | 0.0068 (61.8) | 0.0114 (63.3) | 0.0649 (69.4) | 0.0043 (55.1) | 0.0095 (63.3) | 0.0147 (97.4) |

### Examples 19-24

Table 7 summarizes topical formulations made in accordance with the present invention in a percentage weight-by-weight basis. The formulations were packaged in aluminum tubes with an epoxy phenolic lacquer liner. The formulations of Examples 19-24 were stored at 40°C at 75% relative humidity. At selected time points samples were analyzed for IRM 1, sorbic acid (SA), and BHA content. The results are shown in Table 8 below where the initial values of IRM SA, and BHA are the average of 3 independent determinations, and the values for later time points are from 1 tube. Values are not normalized for weight loss that may have occurred during storage. Test Method 3 was used to determine IRM 1 content. Test Method 4 was used to determine SA and BHA content.

| Table 7 | | | | | | |
|---|---|---|---|---|---|---|
| Ingredient | Ex 19 | Ex 20 | Ex 21 | Ex 22 | Ex 23 | Ex 24 |
| IRM 1 | 0.03 | 0.30 | 0.30 | 0.30 | 0.30 | 0.30 |
| Isostearic acid | 5.00 | 7.00 | 7.00 | 7.00 | 7.00 | 7.00 |
| *Medium-chain Triglycerides | 4.00 | 4.00 | 4.00 | 4.00 | 4.00 | 4.00 |
| CARBOPOL 980 | 1.00 | 1.00 | 1.00 | 1.00 | 1.00 | 1.00 |
| POLOXAMER 188 | 3.50 | 3.50 | 3.50 | 3.50 | 3.50 | 3.50 |
| Propylene glycol | 5.00 | 5.00 | 5.00 | 5.00 | 5.00 | 5.00 |
| Methylparaben | 0.20 | 0.20 | 0.20 | 0.20 | 0.20 | 0.20 |
| Sorbic acid | 0.10 | 0.10 | 0.10 | 0.10 | 0.10 | 0.10 |
| BHA | 0.01 | - | 0.01 | 0.01 | 0.01 | 0.01 |
| Edetate disodium dihydrate | 0.05 | 0.05 | - | 0.05 | 0.03 | 0.01 |
| Sodium hydroxide Solution 20% w/w | 0.80 | 0.80 | 0.80 | 0.80 | 0.80 | 0.80 |
| Purified water | 80.31 | 78.05 | 78.09 | 78.04 | 78.06 | 78.08 |

| | | | | | | |
|---|---|---|---|---|---|---|
| *Caprylic/capric triglyceride available under the trade names CRODAMOL GTCC-PN (Croda, Inc) and MIGLYOL 812N (Sasol). | | | | | | |

| Table 8 | | | | | | |
|---|---|---|---|---|---|---|
| Ingredient - Time Point | Content (% w/w) (% Initial) | | | | | |
| | Ex 19 | Ex 20 | Ex 21 | Ex 22 | Ex 23 | Ex 24 |
| IRM 1 - 0 month | 0.0298 (100.0) | 0.303 (100.0) | 0.303 (100.0) | 0.301 (100.0) | 0.302 (100.0) | 0.303 (100.0) |
| IRM 1 - 2 month | 0.0299 (100.3) | 0.300 (99.0) | 0.306 (101.0) | 0.303 (100.7) | 0.306 (101.3) | 0.305 (100.7) |
| IRM 1 - 4 month | 0.0299 (100.3) | 0.300 (99.0) | 0.304 (100.3) | 0.304 (101.0) | 0.304 (100.7) | 0.305 (100.7) |
| IRM 1 - 6 month | 0.0299 (100.3) | 0.296 (97.7) | 0.302 (99.7) | 0.305 (101.3) | 0.306 (101.3) | 0.306 (101.0) |
| SA - 0 month | 0.0996 (100.0) | 0.0966 (100.0) | 0.0995 (100.0) | 0.0997 (100.0) | 0.1005 (100.0) | 0.1007 (100.0) |
| SA - 2 month | 0.0983 (98.7) | 0.0909 (94.1) | 0.0980 (98.5) | 0.0994 (99.7) | 0.1000 (99.5) | 0.0996 (98.9) |
| SA - 4 month | 0.1003 (100.7) | 0.0825 (85.4) | 0.0976 (98.1) | 0.0996 (99.9) | 0.0993 (98.8) | 0.0988 (98.1) |
| SA - 6 month | 0.0996 (100.0) | 0.0756 (78.3) | 0.0969 (97.4) | 0.0997 (100.0) | 0.0997 (99.2) | 0.0999 (99.2) |
| BHA - 0 month | 0.0095 (100) | *ND | 0.0097 (100) | 0.0098 (100) | 0.0100 (100) | 0.0099 (100) |
| BHA - 2 month | 0.0092 (97) | ND | 0.0085 (88) | 0.0098 (100) | 0.0100 (100) | 0.0099 (100) |
| BHA - 4 month | 0.0096 (101) | ND | 0.0083 (86) | 0.0101 (103) | 0.0101 (101) | 0.0094 (95) |
| BHA - 6 month | 0.0094 (99) | ND | 0.0076 (78) | 0.0101 (103) | 0.0102 (102) | 0.0100 (101) |

| | | | | | | |
|---|---|---|---|---|---|---|
| *ND = not determined | | | | | | |

Table 9 summarizes topical formulations made in accordance with the present invention in a percentage weight-by-weight basis. The formulations were packaged in aluminum tubes with an epoxy phenolic lacquer liner.

| Table 9 | | | | | |
|---|---|---|---|---|---|
| Ingredient | Ex 25 | Ex 26 | Ex 27 | Ex 28 | Ex 29 |
| IRM 1 | 0.01 | 0.05 | 0.10 | 0.10 | 0.10 |
| Isostearic acid | 5.00 | 5.00 | 5.00 | 5.00 | 5.00 |
| *Medium-chain Triglycerides | 4.00 | 4.00 | 4.00 | 4.00 | 4.00 |
| CARBOPOL 980 | 1.00 | 1.00 | 1.00 | 1.00 | 1.00 |
| POLOXAMER 188 | 3.50 | 3.50 | 3.50 | 3.50 | 3.50 |
| Propylene glycol | 5.00 | 5.00 | 5.00 | 5.00 | 5.00 |
| Methylparaben | 0.20 | 0.20 | 0.20 | 0.20 | 0.20 |
| Sorbic acid | 0.10 | 0.10 | 0.10 | 0.10 | 0.10 |
| BHA | 0.01 | 0.01 | 0.01 | 0.01 | 0.01 |
| Edetate disodium dihydrate | 0.05 | 0.05 | 0.05 | 0.05 | 0.05 |
| Sodium hydroxide Solution 20% w/w | 0.80 | 0.80 | 0.80 | 0.40 | 1.20 |
| Purified water | 8033 | 80.29 | 80.24 | 80.64 | 79.84 |

| | | | | | |
|---|---|---|---|---|---|
| *Caprylic/capric triglyceride available under the trade names CRODAMOL GTCC-PN (Croda, Inc) and MIGLYOL 812N (Sasol). | | | | | |

Various modifications and alterations to this invention will become apparent to those skilled in the art without departing from the scope of the present invention which is defined by the claims. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A topical formulation comprising:
an imidazonaphthyridine amine immune response modifier (IRM) compound;
a preservative system comprising a sorbic acid preservative selected from the group consisting of sorbic acid, esters thereof, salts thereof, and combinations thereof; and
an antioxidant.

2. The topical formulation of claim 1 further comprising a fatty acid.

3. The topical formulation of claim 1 or claim 2 further comprising a hydrophobic, aprotic component miscible with a fatty acid and comprising a hydrocarbyl group of 7 or more carbon atoms.

4. The topical formulation of any one of claims 1 through 3 wherein the antioxidant is selected from the group consisting of ascorbic acid, ascorbyl palmitate, butylated hydroxyanisole, butylated hydroxytoluene, cysteine, propyl gallate, sodium formaldehyde sulfoxylate, tocopherol, and combinations thereof.

5. The topical formulation of any one of claims 1 through 4 wherein the preservative system comprises sorbic acid, isopropyl sorbate, calcium sorbate, potassium sorbate, sodium sorbate, triethanolamine sorbate, or combinations thereof.

6. The topical formulation of any one of claims 1 through 5 wherein the preservative system further includes a preservative enhancing solubilizer.

7. The topical formulation of any one of claims 1 through 6 further comprising a chelating agent.

8. The topical formulation of any one of claims I through 7 further comprising a hydrophilic viscosity enhancing agent.

9. The topical formulation according to claim 1 comprising:
0.001% by weight to 5.0% by weight of an imidazonaphthyridine amine immune response modifier (IRM) compound;
a preservative system comprising:
0.02% by weight to 0.2% by weight of a sorbic acid preservative selected from the group consisting of sorbic acid, esters thereof, salts thereof, and combinations thereof;
0 to 10.0% by weight of a preservative enhancing solubilizer; and
0.05% by weight to 0.2% by weight of a secondary preservative compound;
0.001% by weight to 0.2% by weight of an antioxidant comprising hydrogen atom donating functionality;
0 to 0.1% by weight of a chelating agent;
1% by weight to 30% by weight of a fatty acid;
1% by weight to 15% by weight of a medium-chain triglyceride;
0.2% by weight to 2.0% by weight of a viscosity enhancing agent;
0.1% by weight to 6.0% by weight of an emulsifier; and
water;
wherein the formulation has a pH of 4.0 to 6.0 and the weight percentages are based on the total weight of the formulation.

10. The topical formulation according to claim 1 comprising:
0.001% by weight to 5.0% by weight of an imidazonaphthyridine amine;
0.02% by weight to 0.2% by weight of a sorbic acid preservative selected from the group consisting of sorbic acid, esters thereof, salts thereof, and combinations thereof;
0 to 10.0% by weight of propylene glycol;
0.05% by weight to 0.2% by weight of methylparaben;
0.001% by weight to 0.2% by weight of butylated hydroxyanisole, butylated hydroxytoluene, or combinations thereof;
0 to 0.1% by weight of ethylenediaminetetraacetic acid, a hydrate thereof, a salt thereof, a hydrate of a salt thereof, or combinations thereof;
1% by weight to 30% by weight of isostearic acid;
1% by weight to 15% by weight of a medium-chain triglyceride;
0.2% by weight to 2.0% by weight of a carbomer;
0.1% by weight to 6.0% by weight of a poloxamer; and
water;
wherein the formulation has a pH of 4.0 to 6.0 and the weight percentages are based on the total weight of the formulation.

11. The topical formulation of claim 10 wherein the imidazonaphthyridine amine is 2-methyl-1-(2-methylpropyl)-1*H*-imidazo[4,5-*c*][1,5]naphthyridin-4-amine.

12. The topical formulation of any of claims 1 to 9 wherein the imidazonaphthyridine amine is 2-methyl-1-(2-methylpropyl)-1*H*-imidazo[4,5-*c*][1,5]naphthyridin-4-amine.

## Patentansprüche

1. Topische Formulierung, umfassend:
eine Imidazonaphthyridinamin-Immunreaktionsmodifikator-(IRM-)Verbindung;
ein Konservierungssystem, umfassend ein Sorbinsäure-Konservierungsmittel, das aus der Gruppe bestehend aus Sorbinsäure, deren Estern, deren Salzen und Kombinationen derselben ausgewählt ist; und
ein Antioxidationsmittel.

2. Topische Formulierung nach Anspruch 1, ferner umfassend eine Fettsäure.

3. Topische Formulierung nach Anspruch 1 oder Anspruch 2, ferner umfassend eine hydrophobe, aprotische Komponente, die mit einer Fettsäure mischbar ist und eine Kohlenwasserstoffgruppe mit 7 oder mehr Kohlenstoffatomen umfasst.

4. Topische Formulierung nach einem der Ansprüche 1 bis 3, wobei das Antioxidationsmittel aus der Gruppe bestehend aus Ascorbinsäure, Ascorbylpalmitat, butyliertem Hydroxyanisol, butyliertem Hydroxytoluol, Cystein, Propylgallat, Natriumformaldehydsulfoxylat, Tocopherol und Kombinationen derselben ausgewählt ist.

5. Topische Formulierung nach einem der Ansprüche 1 bis 4, wobei das Konservierungssystem Sorbinsäure, Isopropylsorbat, Calciumsorbat, Kaliumsorbat, Natriumsorbat, Triethanolaminsorbat oder Kombinationen derselben umfasst.

6. Topische Formulierung nach einem der Ansprüche 1 bis 5, wobei das Konservierungssystem des Weiteren einen konservierungsverbessernden Löslichmacher einschließt.

7. Topische Formulierung nach einem der Ansprüche 1 bis 6, ferner umfassend einen Chelatbildner.

8. Topische Formulierung nach einem der Ansprüche 1 bis 7, ferner umfassend ein hydrophiles viskositätserhöhendes Mittel.

9. Topische Formulierung nach Anspruch 1, umfassend:
0,001 Gew.-% bis 5,0 Gew.-% einer Imidazonaphthyridinamin-Immunreaktionsmodifikator- (IRM-)Verbindung;
ein Konservierungssystem, umfassend:
0,02 Gew.-% bis 0,2 Gew.-% eines Sorbinsäure-Konservierungsmittels, das aus der Gruppe bestehend aus Sorbinsäure, deren Estern, deren Salzen und Kombinationen derselben ausgewählt ist;
0 bis 10,0 Gew.-% eines konservierungsverbessernden Löslichmachers; und
0,05 Gew.-% bis 0,2 Gew.-% einer sekundären konservierenden Verbindung;
0,001 Gew.-% bis 0,2 Gew.-% eines Antioxidationsmittels, das eine Wasserstoffatom-Donorfunktionalität umfasst;
0 bis 0,1 Gew.-% eines Chelatbildners;
1 Gew.-% bis 30 Gew.-% einer Fettsäure;
1 Gew.-% bis 15 Gew.-% eines mittelkettigen Triglycerids;
0,2 Gew.-% bis 2,0 Gew.-% eines viskositätserhöhenden Mittels;
0,1 Gew.-% bis 6,0 Gew.-% eines Emulgators; und
Wasser;
wobei die Formulierung einen pH-Wert von 4,0 bis 6,0 aufweist und die Gewichtsprozente auf das Gesamtgewicht der Formulierung bezogen sind.

10. Topische Formulierung nach Anspruch 1, umfassend:
0,001 Gew.-% bis 5,0 Gew.-% eines Imidazonaphthyridinamins;
0,02 Gew.-% bis 0,2 Gew.-% eines Sorbinsäure-Konservierungsmittels, das aus der Gruppe bestehend aus Sorbinsäure, deren Estern, deren Salzen und Kombinationen derselben ausgewählt ist;
0 bis 10,0 Gew.-% Propylenglycol;
0,05 Gew.-% bis 0,2 Gew.-% Methylparaben;
0,001 Gew.-% bis 0,2 Gew.-% butyliertes Hydroxyanisol, butyliertes Hydroxytoluol oder Kombinationen derselben;
0 bis 0,1 Gew.-% Ethylendiamintetraessigsäure, eines Hydrats derselben, eines Salzes derselben, eines Hydrats oder eines Salzes derselben oder von Kombinationen derselben;
1 Gew.-% bis 30 Gew.-% Isostearinsäure;
1 Gew.-% bis 15 Gew.-% eines mittelkettigen Triglycerids;
0,2 Gew.-% bis 2,0 Gew.-% eines Carbomers;
0,1 Gew.-% bis 6,0 Gew.-% eines Poloxamers; und
Wasser;
wobei die Formulierung einen pH-Wert von 4,0 bis 6,0 aufweist und die Gewichtsprozente auf das Gesamtgewicht der Formulierung bezogen sind.

11. Topische Formulierung nach Anspruch 10, wobei das Imidazonaphthyridinamin 2-Methyl-1-(2-methylpropyl)-1*H-*imidazo[4,5-c][1,5]naphthyridin-4-amin ist.

12. Topische Formulierung nach einem der Ansprüche 1 bis 9, wobei das Imidazonaphthyridinamin 2-Methyl-1-(2-methylpropyl)-1*H-*imidazo[4,5-c][1,5]naphthyridin-4-amin ist.

## Revendications

1. Formulation topique comprenant :
un composé modificateur de réponse immunitaire (IRM) imidazonaphthyridine amine ;
un système conservateur comprenant un conservateur à base d'acide sorbique choisi dans le groupe constitué de l'acide sorbique, de ses esters, de ses sels et de combinaisons de ceux-ci ; et
un antioxydant.

2. Formulation topique selon la revendication 1, comprenant en outre un acide gras.

3. Formulation topique selon la revendication 1 ou la revendication 2, comprenant en outre un composant hydrophobe, aprotique miscible avec un acide gras et comprenant un groupe hydrocarbyle de 7 ou plus d'atomes de carbone.

4. Formulation topique selon l'une quelconque des revendications 1 à 3, dans laquelle l'antioxydant est choisi dans le groupe constitué de l'acide ascorbique, du palmitate d'ascorbyle, de l'hydroxyanisole butylé, de l'hydroxytoluène butylé, de la cystéine, du gallate de propyle, du formaldéhyde sulfoxylate de sodium, du tocophérol, et de combinaisons de ceux-ci.

5. Formulation topique selon l'une quelconque des revendications 1 à 4, dans laquelle le système conservateur comprend de l'acide sorbique, du sorbate d'isopropyle, du sorbate de calcium, du sorbate de potassium, du sorbate de sodium, du sorbate de triéthanolamine, ou des combinaisons de ceux-ci.

6. Formulation topique selon l'une quelconque des revendications 1 à 5, dans laquelle le système conservateur comporte en outre un solubilisant améliorant le conservateur.

7. Formulation topique selon l'une quelconque des revendications 1 à 6, comprenant en outre un agent chélatant.

8. Formulation topique selon l'une quelconque des revendications 1 à 7, comprenant en outre un agent hydrophile améliorant la viscosité.

9. Formulation topique selon la revendication 1, comprenant:
de 0,001 % en poids à 5,0 % d'un composé modificateur de réponse immunitaire (IRM) imidazonaphthyridine amine ;
un système conservateur comprenant :
de 0,02 % en poids à 0,2 % en poids d'un conservateur à base d'acide sorbique choisi dans le groupe constitué de l'acide sorbique, de ses esters, de ses sels, et de combinaisons de ceux-ci ;
de 0 à 10,0 % en poids d'un solubilisant améliorant le conservateur ; et
de 0,05 % en poids à 0,2 % en poids d'un composé conservateur secondaire ;
de 0,001 % en poids à 0,2 % en poids d'un antioxydant comprenant une fonctionnalité de donneur d'atome d'hydrogène ;
de 0 à 0,1 % en poids d'un agent chélatant ;
de 1 % en poids à 30 % en poids d'un acide gras ;
de 1 % en poids à 15 % en poids d'un triglycéride de chaîne moyenne ;
de 0,2 % en poids à 2,0 % en poids d'un agent améliorant la viscosité ;
de 0,1% en poids à 6,0 % en poids d'un émulsifiant ; et
de l'eau ;
où la formulation a un pH de 4,0 à 6,0 et les pourcentages en poids sont basés sur le poids total de la formulation.

10. Formulation topique selon la revendication 1, comprenant :
de 0,001% en poids à 5,0% en poids d'une imidazonaphthyridine amine ;
de 0,02 % en poids à 0,2 % en poids d'un conservateur à base d'acide sorbique choisi dans le groupe constitué de l'acide sorbique, de ses esters, de ses sels, et de combinaisons de ceux-ci ;
de 0 à 10,0 % en poids de propylène glycol ;
de 0,05 % en poids à 0,2% en poids de méthylparabène ;
de 0,001 % en poids à 0,2 % en poids d'hydroxyanisole butylé, d'hydroxytoluène butylé, ou de combinaisons de ceux-ci ;
de 0 à 0,1 % en poids d'acide éthylènediaminetétraacétique, on un de ses hydrates, un de ses sels, un hydrate d'un de ses sels, ou des combinaisons de ceux-ci ;
de 1 % en poids à 30 % en poids d'acide isostéarique ;
de 1 % en poids à 15 % en poids d'un triglycéride de chaîne moyenne ;
de 0,2 % en poids à 2,0 % en poids d'un carbomère ;
de 0,1 % en poids à 6,0 % en poids d'un poloxamère ; et
de l'eau ;
où la formulation a un pH de 4,0 à 6,0 et les pourcentages en poids sont basés sur le poids total de la formulation.

11. Formulation topique selon la revendication 10, dans laquelle l'imidazonaphthyridine amine est la 2-méthyl-1-(2-méthylpropyl)-1*H*-imidazo[4,5-*c*][1,5]naphthyridin-4-amine.

12. Formulation topique selon l'une quelconque des revendications 1 à 9, dans laquelle l'imidazonaphthyridine amine est la 2-méthyl-1-(2-méthylpropyl)-1*H*-imidazo[4,5-*c*][1,5]naphthyridin-4-amine.
